# EUROPÄISCHE PATENTSCHRIFT

(11) **EP 0 608 388 B1**
(45) Veröffentlichungstag und Bekanntmachung des Hinweises auf die Patenterteilung: **08.07.1998**
(21) Anmeldenummer: 93908759.9
(22) Anmeldetag: 12.10.1992
(51) Int. Cl.: C09C 1/00

(54) **GEFÄRBTE UND BESCHICHTETE PLÄTTCHENFÖRMIGE PIGMENTE**
COLOURED AND COATED PLATELIKE PIGMENTS
PIGMENTS EN PLAQUETTES ENROBES ET COLORES

(30) Priorität: 18.10.1991 DE 4134600; 22.11.1991 DE 4138376; 10.04.1992 DE 4212119; 09.05.1992 DE 4215276
(43) Veröffentlichungstag der Anmeldung: 03.08.1994
(73) Patentinhaber: MERCK PATENT GmbH, 64271 Darmstadt (DE)
(72) Erfinder: BAUER, Gerd, D-8752 Kleinostheim (DE); OSTERRIED, Karl, D-6110 Dieburg (DE); SCHMIDT, Christoph, D-6233 Kelkheim (DE); VOGT, Reiner, D-6102 Pfungstadt (DE); KNIESS, Helge-Bettina, D-6109 Weiterstadt (DE); UHLIG, Michael, D-6108 Weiterstadt 3 (DE); SCHÜL, Norbert, D-6148 Heppenheim (DE); BRENNER, Günter, D-6103 Griesheim (DE)
(86) Internationale Anmeldenummer: EP9202351
(87) Internationale Veröffentlichungsnummer: WO9308237

(56) Entgegenhaltungen:
- EP-A- 0 266 248
- EP-A- 0 484 108
- FR-A- 1 273 230
- FR-A- 1 581 309

## Beschreibung

Die Erfindung betrifft plättchenförmige Pigmente mit hohem Glanz und hohem Deckvermögen oder hoher Transparenz.

Deckvermögen und Glanz sind bei plättchenförmigen Pigmenten oftmals nur schwer gleichzeitig in befriedigendem Ausmaß zu realisieren. So zeichnen sich etwa mit einer oder mehreren dünnen Metalloxidschichten belegte Glimmerplättchen durch Interferenzfarben und einen hohen Glanz, gleichzeitig aber auch wegen des durchsichtigen Substrates durch eine hohe Transparenz und damit ein vergleichsweise geringes Deckvermögen aus. Das Deckvermögen kann durch Verwendung farbiger Metalloxidschichten aus z.B. Chromoxid oder Eisenoxid zwar verbessert werden, jedoch genügt auch das Deckvermögen derartiger Pigmente häufig nicht allen Anforderungen. Das Deckvermögen kann weiter erhöht werden, wenn eine rauhe Substratoberfläche vorhanden ist, oder wenn relativ rauhe Metalloxidschichten abgeschieden werden: durch die erhöhte Zahl an Streuzentren nimmt das Deckvermögen zu, der Glanz jedoch ab.

Die Dicke der Metalloxidschichten liegt je nach Brechzahl des verwendeten Metalloxids und nach der gewünschten Interferenzfarbe typischerweise zwischen 50 und 250 nm. Da es an der Phasengrenze von Materialien mit unterschiedlichen optischen Brechungsindizes zu Reflexionen kommt, beobachtet man bei paralleler Ausrichtung der plättchenförmigen Pigmente im reflektierten Licht Interferenzeffekte, die vom Beobachtungswinkel und den optischen Dicken der Metalloxidschichten abhängen; im durchgehenden Licht sieht man bei nicht absorbierenden Materialien die entsprechende Komplementärfarbe.

Voraussetzung für das Zustandekommen derartiger Interferenzeffekte ist eine glatte Substratoberfläche.

Bisher sind für die Herstellung von Interferenzpigmenten als plättchenförmige Substrate überwiegend natürliche Materialien wie z.B. Glimmer verwendet worden. Da es sich um ein natürlich vorkommendes Material handelt, ist die Oberfläche derartiger Substrate nicht ideal glatt, sondern weist Unregelmäßigkeiten wie zum Beispiel Stufen auf, wodurch die Qualität der resultierenden Interferenzpigmente limitiert ist.

Ein weiterer Nachteil von natürlichen Materialien, wie zum Beispiel Glimmer sind die Verunreinigungen durch färbende Fremdionen, beispielsweise Eisenionen, die sich negativ auf die Farbreinheit des Endproduktes auswirken können.

Es ist weiter vorgeschlagen worden, anstelle von transparentem Glimmer plättchenförmige Metallsubstrate zu verwenden, was zu Pigmenten mit sehr hohem Deckvermögen führt. Andererseits resultiert gleichzeitig ein sehr harter, metallischer Glanz, der oftmals ästhetisch nicht völlig befriedigt. Außerdem besitzen Metallpigmente keine Tiefenwirkung, da das auffallende Licht unmittelbar von der Oberfläche reflektiert wird.

Als synthetisches Material sind dünne Glasplättchen vorgeschlagen worden, die durch Walzen einer Glasschmelze mit nachfolgendem Mahlen erhalten werden. Interferenzpigmente auf der Basis derartiger Materialien weisen zwar Farbeffekte auf, die denen herkömmlicher, auf Glimmer basierender Pigmente überlegen sind. Nachteilig ist jedoch, daß die Glasplättchen eine sehr große mittlere Dicke von etwa 10-15 µm und eine sehr breite Dickenverteilung (typischerweise zwischen 4 und 20 µm) aufweisen, während die Dicke von Interferenzpigmenten typischerweise nicht größer ist als 3 µm. In EP 0,384,596 wird ein Verfahren beschrieben, bei dem hydratisiertes Alkalisilikat bei Temperaturen von 480-500 °C mit einem Luftstrahl beaufschlagt wird, wobei sich Blasen mit dünnen Wandstärken bilden; die Blasen werden anschießend zerkleinert und man erhält plättchenförmige Alkalisilikatsubstrate mit einer Dicke von weniger als 3 µm. Das Verfahren ist jedoch aufwendig und die Dickenverteilung der erhaltenen Plättchen ist relativ breit.

In EP 0,240,952 ist ein kontinuierliches Bandverfahren zur Herstellung verschiedener plättchenförmiger Materialien, darunter auch Siliciumdioxid vorgeschlagen worden. Dabei wird über ein Rollensystem auf ein glattes Band ein dünner flüssiger Film definierter Dicke eines Precursors des plättchenförmigen Materials aufgebracht; der Film wird getrocknet und von dem Band abgelöst, wobei sich plättchenförmige Teilchen bilden. Die Teilchen werden anschließend ggf. geglüht, ggf. gemahlen und klassiert.

Als Precursormaterialien werden metallorganische Verbindungen (Alkoholate), wie z.B. Tetraethylorthosilikat, verwendet. Der Film wird durch Trocknen polymerisiert und vom Band mit Hilfe eines Schabers abgekratzt, wobei kleine Plättchen erhalten werden; diese werden dann anschließend zur Umwandlung in das entsprechende Metalloxid bei Temperaturen von 500 °C geglüht. Daneben wird als Precursor ein ggf. in Methanol dispergiertes Metalloxidsol verwendet, welches in analoger Weise als Film aufgebracht, getrocknet und geglüht wird.

Nachteilig sind jedoch die Verwendung sehr teurer Precursormaterialien sowie insbesondere die erhöhten Anforderungen an die Arbeitsplatzsicherheit, die beim Einsatz metallorganischer Verbindungen gestellt werden müssen. Die vollständige chemische Umwandlung des Precursors in das gewünschte Schichtmaterial macht in der Regel eine starke Erhitzung des Filmes und des Bandmaterials erforderlich. Neben der dabei auftretenden erheblichen thermischen Belastung des Bandmaterials, wirken sich auch der hohe Energieaufwand und die Einschränkung der Prozeßgeschwindigkeit sehr nachteilig auf die Wirtschaftlichkeit des Verfahrens aus.

Auch die in EP 0,236,952 beschriebene Verwendung von wäßrigen Oxid- bzw. Hydroxidsolen ist problematisch, da die entstehenden Filme nicht homogen sind sondern aus ungleichmäßig großen Partikeln aufgebaut sind. Dies macht eine Behandlung bei sehr hohen Temperaturen erforderlich, um dem Material die notwendige Homogenität, Formgenauigkeit und Festigkeit zu geben.

In US 3 138 475 ist ein kontinuierliches Bandverfahren zur Herstellung plättchen- oder flitterartiger Oxyde oder Oxydhydrate von Metallen der IV. und V. Gruppe sowie der Eisen-Gruppe des Periodensystems beschrieben. Dabei wird auf ein umlaufendes Band ggf. zunächst eine Trennschicht aus z.B. Siliconlack aufgebracht, um das spätere Ablösen der Metalloxidschicht zu erleichtern. Anschließend wird ein Flüssigkeitsfilm aus einer Lösung einer hydrolysierbaren Verbindung des in das gewünschte Oxid umzuwandelnden Metalls aufgebracht, der Film wird getrocknet und anschließend mit einer Rüttelvorrichtung abgelöst. Es wird zwar erwähnt, daß nach diesem Verfahren auch SiO₂-Plättchen hergestellt werden können, wobei jedoch das Verfahren nur ganz allgemein beschrieben wird und kein konkretes Beispiel angeführt ist.

In JP 64-9803 wird ein Verfahren zur Herstellung eines plättchenförmigen Metalloxids mit dispergierten Feinpartikeln eines zweiten Metalloxids mit höherer Brechzahl auf einem endlosen Band beschrieben. Die nach diesem Verfahren erhaltenen Produkte, die als Lichtschutzfilter in der Kosmetik verwendet werden, bestehen zum Beispiel aus einer Matrix aus Siliciumdioxid, in der feine Partikel aus Titandioxid dispergiert sind. Dieses Produkt zeigt aber keine Interferenzfarben, weil die als Streuzentren wirkenden Titandioxidpartikel nicht gleichmäßig auf einer glatten Fläche verteilt sind.

Aus JP 2-32 170 ist ein Pigment bekannt, das aus einem Basismaterial, zum Beispiel einem Titandioxid-Glimmerpigment, besteht, wobei auf eine erste Interferenzschicht aus Titandioxid kolloidale Metallpartikel (Silber) aufgesputtert sind.

Als Deckschicht ist dann wiederum Titandioxid als zweite Interferenzschicht aufgebracht. Dieses Pigment hat den Nachteil, daß kein Weiß erzeugt werden kann, da die Metallpartikel absorbieren und damit das Produkt dunkel färben. Außerdem ist die Herstellung sehr teuer, da vier verschiedene Schichten mit Hilfe unterschiedlicher Verfahren aufgebracht werden müssen.

Ein Pigment mit ähnlichem Aufbau ist aus EP-A-0 484 108 bekannt. Auf ein Titandioxid-Glimmerpigment als Basismaterial wird Titan durch Sputtern aufgebracht. Ein Teil des Titans reduziert das Titandioxid des Basismaterials zu Suboxiden, die als lichtabsorbierende Bereiche auf der Pigmentoberfläche wirken. Die unverändert gebliebenen Titanpartikel verleihen dem Pigment metallischen Glanz.

Abgesehen von den hohen Herstellungskosten können nach diesem Verfahren nur dunkle Pigmente von geringer Transparenz hergestellt werden. Außerdem können keine Interferenzfarben erzeugt werden.

Aufgabe der vorliegenden Erfindung ist es, plättchenförmige Interferenzpigmente mit hohem Glanz und hohem Deckvermögen oder hoher Transparenz bereitzustellen, die mit Hilfe eines einfachen und wirtschaftlichen Verfahrens herstellbar sind.

Diese Aufgabe wird gemäß der vorliegenden Erfindung gelöst durch die Bereitstellung eines plättchenförmigen Pigmentes mit hohem Glanz und hohem Deckvermögen oder hoher Transparenz, bestehend aus einer durch Verfestigung eines flüssigen Precursors hergestellten transparenten, anorganischen, plättchenförmigen Matrix, die zur Erzielung des Glanzes mit einer oder mehreren dünnen, transparenten oder semitransparenten reflektierenden Schichten aus Metalloxiden oder Metallen belegt ist, dadurch erhältlich, daß
- der Precursor des Matrixmaterials als dünner Film auf ein endloses Band aufgebracht wird,
- der flüssige Film durch Trocknung verfestigt wird,
- im verfestigten Film die Matrix durch eine chemische Reaktion aus dem Precursor entwickelt wird,
- der durch Trocknung verfestigte Film anschließend mit einer Säure behandelt wird,
- der Film anschließend vom Trägermedium getrennt und gewaschen wird,
- die Partikel gegebenenfalls getrocknet, geglüht, gemahlen und klassiert werden und
- die erhaltenen Filmpartikel mit einer oder mehreren Schichten aus Metalloxiden oder Metallen belegt werden.

Weiterhin wird diese Aufgabe gemäß der Erfindung gelöst durch ein Verfahren zur Herstellung der erfindungsgemäßen Pigmente, bestehend aus einer durch Verfestigung eines flüssigen Precursors hergestellten transparenten, anorganischen, plättchenförmigen Matrix, die mit einer oder mehreren dünnen, transparenten oder semitransparenten Schichten aus Metalloxiden oder Metallen belegt ist, wobei
- ein Precursor des Matrixmaterials als dünner Film auf ein endloses Band aufgebracht wird,
- der flüssige Film durch Trocknung verfestigt wird,
- im verfestigten Film die Matrix durch eine chemische Reaktion aus dem Precursor entwickelt wird,
- die entstandene Schicht anschließend vom Trägermedium getrennt und gewaschen wird und
- die Partikel gegebenenfalls getrocknet, geglüht, gemahlen und klassiert werden, das
dadurch gekennzeichnet ist, daß der durch Trocknung verfestigte Film anschließend mit einer Säure behandelt wird und die erhaltenen Filmpartikel mit einer oder mehreren reflek-tierenden Schichten aus Metalloxiden oder Metallen belegt werden.

Zweckmäßige Ausgestaltungen sind in den Unteransprüchen angegeben.

Gegenstand der Erfindung ist außerdem die Verwendung der erfindungsgemäß hergestellten Pigmente in Formulierungen wie Lacken, Druckfarben, Kosmetika oder Kunststoffen oder als Korrosionsschutzmittel.

Die erfindungsgemäßen Pigmente basieren auf einer plättchenförmigen, transparenten Matrix, die durch Netzwerkbildner bzw. Netzwerkwandler, wie zum Beispiel Aluminiumoxid, Boroxid oder Phosphoroxid, Natriumoxid, Lithiumoxid, Kaliumoxid oder Kalziumoxid modifiziert sein kann. Die Matrix kann z.B. aus Siliziumdioxid, Silikaten, Boroxid, Boraten, Aluminiumoxid, Aluminaten oder anderen transparenten, stabilen und zur Aufnahme von löslichen oder unlöslichen Farbmitteln befähigten Materialien bestehen. Die plättchenförmigen Matrixpartikel haben typischerweise eine Dicke zwischen 0,05 und 5 µm und insbesondere zwischen 0,2 und 2,0 µm. Die Ausdehnung in den beiden anderen Dimensionen beträgt üblicherweise zwischen 1 und 250 µm und insbesondere zwischen 2 und 100 µm.

Als Ausgangsmaterial (Precursor) für die Herstellung der Matrix werden Lösungen von anorganischen oder organischen Verbindungen der Metalle Aluminium, Silicium, Kalium oder Natrium mit beispielsweise Boraten, Aluminaten, Poly- bzw. Metaphosphaten, Silikaten oder Gemischen derselben eingesetzt. Ein bevorzugter Precursor ist Wasserglas.

In die Matrix sind als unlösliche Farbmittel Pigmentpartikel, deren Abmessungen deutlich kleiner sind als die der Matrix, im allgemeinen dreidimensional regellos eingelagert. Es handelt sich um sphärische oder dreidimensional unregelmäßig geformte Partikel mit einer maximalen Ausdehnung von weniger als 3 µm und insbesondere von weniger als 1 µm, wobei noch kleinere Pigmente vielfach bevorzugt sind. Agglomerate von käuflichen Pigmenten, die eine zu große Ausdehnung haben können, werden vorzugsweise in einer Kugelmühle, in einer Sandmühle oder in einer ähnlichen Vorrichtung zerkleinert. Es können jedoch auch manchmal größere Pigmentpartikel verwendet werden, wobei jedoch die mittlere Größe der Pigmentpartikel in jedem Fall kleiner sein sollte als die mittlere Dicke der Matrix, um die Ausbildung glatter, dünner, glanzerzeugender Schichten zu ermöglichen. Der Begriff Pigmentpartikel ist hier weit zu verstehen und umfaßt Weiß-, Schwarz-, Buntsowie Leuchtpigmente.

Geeignete anorganische Pigmentpartikel sind z.B. Weißpigmente wie z.B. Titandioxid, Bariumsulfat oder Zinkoxid, Schwarzpigmente wie z.B. Magnetit oder Pigmentruß und auch Buntpigmente wie z.B. Eisen- oder Chromoxid, Mischphasenoxide wie z.B. (Ti, Cr, Sb)O₂, CoAl₂O₄ (Thenards Blau), ZnAl₂O₄ (Rinmans Grün) (Fe, Cr)₂O₃, weiter Sulfide wie z.B. CdS und andere anorganische Buntpigmente. Geeignet sind auch anorganische Leuchtpigmente wie z.B. fluoreszierendes silberdotiertes Zinkoxid, phosphoreszierendes kupferdotiertes Zinksulfid oder Ultramarinpigmente.

Geeignete organische Pigmente sind Azopigmente, Anthrchinon-Pigmente, Indigo- oder Thioindigo-Derivate, Diketo-Pyrrolo-Pyrrol-Pigmente, Perylen-Pigmente oder Phthalocyanin-Pigmente.

Die hier aufgezählten Pigmentpartikel sind ebenso wie Verfahren zu ihrer Herstellung bekannt (s. z.B. H. Kittel, Wissenschaftliche Verlagsgesellschaft, Stuttgart 1960, G. Benzig, Pigmente für Anstrichmittel, Expert Verlag 1988) und sie sind in der Regel auch kommerziell erhältlich. Dabei sind diese Pigmentpartikel jedoch nur beispielhaft zu verstehen, und sie sollen die Erfindung lediglich erläutern, ohne sie in irgendeiner Weise zu begrenzen. Neben den explizit genannten Pigmentpartikeln kann eine Vielzahl weiterer Pigmentpartikel verwendet werden.

In vielen Fällen ist es vorteilhaft, zur besseren Dispergierung der Pigmentpartikel in der Precursorlösung noch Netzmittel zuzusetzen, z.B. nichtionische und/oder ionische handelsübliche Typen. So sind z.B. Polyäthylen- und Polypropylenglykole gut geeignet. Weder der Typ noch die Menge des zugesetzten Netzmittels sind kritisch, aber im allgemeinen liegt der Anteil des Netzmittels maximal bei 2 Gew.-%, bezogen auf die Dispersion.

Die Matrix kann als zusätzlichen Bestandteil auch ein lösliches Farbmittel enthalten. Unter dem Begriff "lösliches Farbmittel" ist entweder ein farbgebendes Metalloxid oder ein löslicher organicher Farbstoff zu verstehen. Dieses lösliche Farbmittel kann entweder als alleiniger Bestandteil oder zusammen mit einem unlöslichen Farbmittel, das heißt mit einem Pigment, in der anorganischen Matrix enthalten sein.

Der lösliche organische Farbstoff ist beispielsweise ein in Lauge löslicher Hydroxyantrachinonfarbstoff oder ein saurer Azofarbstoff.

Zur Verhinderung eines möglichen "Ausblutens" des löslichen Farbmittels aus der Matrix bei der Beschichtung mit einem Metalloxid, kann auf die Matrix zusätzlich eine SiO₂-Schicht aufgebracht werden.

Das farbgebende Metalloxid ist beispielsweise Eisenoxid, Chromoxid oder Kobaltoxid. Generell sind für die Anfärbung der Matrix farbgebende Verbindungen der Metalle, Titan, Vanadium, Chrom, Mangan, Eisen, Kobalt, Nickel und Kupfer, vorzugsweise Verbindungen von Kobalt, Kupfer, Eisen und Chrom geeignet. Sie werden als lösliche Verbindungen dem Precursor des Matrixmaterials zugesetzt.

Man erhält ein farbiges, transparentes Pigment mit einer Farbskala, ähnlich derjenigen von farbigem, transparentem Glas. Durch Zusatz von Eisenverbindungen erhält man zum Beispiel rotbraune Farbtöne, durch Zusatz von Chromverbindungen grüne Farbtöne und durch Zusatz von Kobaltverbindungen blaue Farbtöne.

Das Pigment besitzt eine besonders hohe Transparenz, da infolge der glatten Oberfläche der Plättchen und des Fehlens von lichtstreuenden Partikeln in der Matrix praktisch kein Licht gestreut wird.

Der bereits durch die glatte Oberfläche vorhandene Glanz der Matrixpartikel wird durch das Aufbringen von reflektierenden Schichten beispielsweise aus Metalloxiden, verstärkt. Gleichzeitig werden damit Interferenzfarben erzeugt.

Durch die Kombination aus Glanzwirkung und Absorptionsfarbe lassen sich brilliante Farbeindrücke, beispielsweise für Autolackierungen oder für die Anfärbung von Kunststoffen, realisieren.

Die farbigen Matrixpartikel können auch ohne zusätzliche Beschichtung in verschiedenen Formulierungen eingesetzt werden.

Das lösliche oder unlösliche Farbmittel ist im unbeschichteten Substrat in einem Anteil von 0,01 Gew.% bis 50 Gew.%, vorzugsweise 1 Gew.% bis 30 Gew.% enthalten.

Es wurde gefunden, daß durch Zusatz von Bariumsulfat zur Matrix eine Glättung der Oberfläche der plättchenförmigen Matrixpartikel erfolgt. Bei der nachfolgenden Beschichtung mit Metallen oder Metalloxiden wird dadurch ein höherer Glanz des Pigmentes erreicht.

Das Bariumsulfat mit einer Teilchengröße von 20 nm bis 500 nm, vorzugsweise 100 nm bis 300 nm, wird im Precursor des Matrixmaterials dispergiert. Es können handelsübliche Produkte, beispielsweise BF 10 der Firma Nordmann, Rassmann GmbH & Co. eingesetzt werden.

Der Anteil an Bariumsulfat in unbeschichtetem Substrat beträgt 1 Gew.% bis 50 Gew.%, vorzugsweise 10 Gew.% bis 25 Gew.%.

Die plättchenförmigen Matrixpartikel, die ein unlösliches und/oder lösliches Farbmittel enthalten, sind mit einer oder mehreren dünnen, transparenten oder semitransparenten, reflektierenden Schicht aus Metall oder Metalloxiden belegt, welche zur Glanzerzeugung dienen.

Bevorzugt sind erfindungsgemäße Pigmente, deren Matrix auf mindestens einer Seite mit einer dünnen, halbdurchlässigen Metallschicht überzogen ist. Die Metallschicht weist typischerweise eine Dicke zwischen 5 und 25 nm und insbesondere zwischen 5 und 15 nm auf und besteht z.B. aus Al, Cr, Ag, Au, Cu oder auch anderen Metallen. Die Metallschicht ist sehr glatt und reflektiert spiegelnd - je nach ihrer Dicke - einen größeren oder kleineren Teil des auffallenden Lichts. Das restliche Licht tritt in die Matrix ein und wird, wenn es sich um Absorptionspigmentpartikel handelt, teilweise absorbiert und teilweise an den eingelagerten Pigmentpartikeln gestreut und das restliche Licht durchgelassen. Wenn die erfindungsgemäßen Pigmente z.B. als Bestandteil einer Lackformulierung auf eine Oberfläche aufgebracht werden, ordnen sie sich aufgrund ihrer plättchenförmigen Struktur mehr oder weniger parallel zueinander in übereinanderliegenden Schichten an, und das durch höher liegende Pigmente hindurchtretende Licht wird an darunter liegende Pigmente - wie eben beschrieben - reflektiert, absorbiert, gestreut und durchgelassen. Der durch die beschriebene spezielle Ausgestaltung der erfindungsgemäßen Pigmente hervorgerufene Gesamteffekt ist somit ein in einem weiten Bereich varierbarer, hoher Glanz in Kombination mit der Farbe der eingelagerten Pigmente und einem hohen Deckvermögen, welches auf die Reflexion an der Metalldeckschicht und der Streuung an den eingelagerten Pigmentpartikeln zurückzuführen ist. Die Pigmente können zur Verstärkung des Glanzes auch auf beiden Seiten mit einer dünnen, halbdurchlässigen Metallschicht versehen sein, was besonders bevorzugt ist.

In einer anderen speziellen Ausgestaltung der erfindungsgemäßen Pigmente ist die mit Pigmentpartikeln versehene Matrix mit einer dünnen glatten Metalloxidschicht versehen, wobei der Brechungsindex der Metalloxidschicht größer ist als der Brechungsindex des Matrixmaterials. Geeignete Metalloxide sind z.B. Titandioxid, Zirkondioxid, Zinkdioxid, Eisenoxid und/oder weitere hochbrechende Metalloxide. Die Metalloxidschicht, deren Dicke typischerweise zwischen 20 und 250 nm beträgt, wirkt als Interferenz- bzw. als Glanzschicht und zusätzlich ggf. als Absorptionsschicht, wenn das Metalloxid gefärbt ist. Der Interferenz- bzw. Glanzeffekt kommt dadurch zustande, daß Licht an den Grenzflächen umgebendes Medium/ Metalloxidschicht und Metalloxidschicht/Matrixoberfläche teilweise spiegelnd reflektiert wird, wobei die reflektierten Strahlen miteinander interferieren und bei entsprechender Dicke der Metalloxidschichten Interferenzfarben erzeugen. Dabei handelt es sich wie schon bei den mit einer Metallschicht belegten Matrixpartikeln um einen Multiteilcheneffekt, da das von verschiedenen, parallel ausgerichteten Teilchen reflektierte Licht zur Verstärkung der Interferenzfarbe beiträgt. Der nicht reflektierte Teil des Lichts tritt wiederum in die Matrix ein und wird dort - wie beschrieben - teilweise durchgelassen und teilweise gestreut. Man erhält ein glänzendes und hochdeckendes Pigment, das eine blickwinkelabhängige Interferenzfarbe und die nicht blickwinkelabhängige Farbe der eingelagerten Absorptionspigmentpartikel aufweist, wobei letztere ggf. durch die Absorptionsfarbe der Metalloxidschicht modifiziert sein kann. Zur Verstärkung des Effekts können auch hier beide Seiten des Pigments mit einer Metalloxidschicht belegt sein, was besonders bevorzugt ist.

Pigmente mit einem derartigen "Farbflopp", blickwinkelunabhängige Körperfarbe und blickwinkelabhängige Interferenzfarbe, können beispielsweise in der Drucktechnik für kopiergeschützte Originale eingesetzt werden.

Wird Ruß als unlösliches Farbmittel in die Matrix eingearbeitet, dann bewirkt der schwarze Untergrund, daß das zum Beispiel mit Titandioxid beschichtete Matrixmaterial ein Pigment ergibt, bei welchem die Körperfarbe gleich der Interferenzfarbe ist. Auf diese Weise erhält man eine neue Art von Pigmenten.

Die Beschichtung der plättchenförmigen Matrixpartikel, die ein unlösliches und/oder lösliches Farbmittel enthalten, erfolgt nach bekannten Verfahren gemäß DE 20 09 566, DE 23 13 331, DE 31 51 355 oder DE 32 21 045.

Die Beschichtung von transparentem Matrixplättchen mit hochbrechenden Metalloxiden liefert besonders transparente, hochglänzende Pigmente.

Die Erfindung umfaßt außer den beiden beschriebenen speziellen Ausgestaltungen der erfindungsgemäßen Pigmente auch solche mit komplizierterem Aufbau. So können die Pigmente zur Erzielung besonderer Farbeffekte oder spezieller funktioneller Eigenschaften mit einer oder mehreren weiteren Metalloxidschichten bedeckt sein. Als Beispiel sei angeführt, daß die Pigmente zur Erhöhung der Stabilität in Emaillen und Glasuren mit einer zusätzlichen Deckschicht aus Zinn- oder Cerdioxid versehen sind wie dies in DE 35 35 818 beschrieben ist. Weiterhin kann eine zusätzliche Metalloxidschicht aus z.B. mit Antimonoxid dotiertem Zinnoxid (DE 38 42 330) oder anderen elektrisch leitfähigen Deckschichten den erfindungsgemäßen Pigmenten eine elektrische Leitfähigkeit verleihen. Ein spezieller optischer Effekt kann z.B. erzielt werden, wenn eine nicht gefärbte Metalloxidschicht mit einer gefärbten Metalloxidschicht kombiniert wird, wie dies z.B. in US 3,087,828 vorgeschlagen wird. Erfindungsgemäße Pigmente mit nicht mehr als 2 Metalloxidschichten sind bevorzugt.

Zum Schutz hydrolyseempfindlicher Metallschichten aus z.B. Al können z.B. polymere Schutzschichten etwa aus Polyethylen, Polyacrylaten oder anderen Materialien aufgebracht werden. Weiterhin können Metallschichten auch mit Metalloxidschichten zur Erzielung besonderer Effekte kombiniert werden; so kann etwa eine Schichtfolge Metall/Metalloxid/Metall auf der Matrixoberfläche wie eine besonders effektive Interferenzschicht wirken, wobei die Dicke der Metalloxidschicht die optische Wegdifferenz zwischen den an den Metallschichten reflektierten Strahlen und damit die Interferenzfarbe bestimmt.

Die Dicke der Deckschichten kann in einem weiten Bereich variieren. So liegt die Dicke von halbdurchlässigen Metallschichten typischerweise zwischen 5 und 25 nm, während die Dicke von Metalloxidschichten in der Regel zwischen 20 und 300 nm beträgt. Polymerschutzschichten sind in der Regel nicht dicker als 50 nm. Das Verhältnis aus der Dicke der Matrix zur Dicke der auf einer Seite des erfindungsgemäßen Pigmentes aufgebrachten Schichten liegt zwischen 0,01 und 500 und insbesondere zwischen 0,1 und 150.

Das Deckvermögen und - bei gefärbten Pigmentpartikeln - die beobachtungswinkelunabhängige Absorptionsfarbe der erfindungsgemäßen Pigmente kann in einem weiteren Bereich durch die Konzentration der eingelagerten Pigmentpartikel variiert werden. Der auf das Gewicht des unbeschichteten Substrates bezogene Gewichtsanteil der eingelagerten Pigmentpartikel liegt typischerweise zwischen 0,5 und 40 % und insbesondere zwischen 5 und 25 %. Besonders bevorzugt sind erfindungsgemäße Pigmente mit Weiß- oder Schwarzpigmenten, wobei hier insbesondere Titandioxid- bzw. Rußpartikel verwendet werden.

Die Farbe von Pigmenten, welche eingelagerte Rußpartikel enthalten, kann je nach der Konzentration der Rußpartikel z.B. von altweiß über hellgrau, metallfarbig, dunkelgrau bis hin zu schwarz reichen. Besonders interessant sind metallfarbige und insbesondere aluminiumfarbige Pigmente. Da diesen Pigmenten durch die nachfolgende Beschichtung mit dünnen transparenten Metalloxidschichten aus z.B. Titan-, Zirkonium-, Zinn- oder Zinkoxid Glanz verliehen wird, können sie plättchenförmige Metallpigmente z.B. in Wasserlackformulierungen ersetzen, wo Metallpigmente und insbesondere Aluminiumpigmente wegen der Korrosionsprobleme (Wasserstoffentwicklung) nur mit Schutzschichten z.B. aus organischen Polymeren eingesetzt werden können. Darüberhinaus kommt es auch bei korrosionsgeschützten Metallpigmenten oftmals zu Problemen, da die organische Schutzschicht mechanisch verletzt ("angeritzt") werden kann. Die erfindungsgemäßen, metallfarbigen, glänzenden Pigmente, welche Ruß in eine transparente Matrix eingelagert enthalten, sind chemisch und mechanisch außerordentlich stabil und sie sind von hoher Brillianz und von hohem ästhetischen Reiz. Sie sind daher besonders als Ersatz metallischer Pigmente bei verschiedensten Anwendungen, insbesondere für auf Wasser basierende Formulierungen, geeignet.

Bevorzugt sind weiter auch erfindungsgemäße Pigmente mit einem oder mehreren organischen oder anorganischen und insbesondere anorganischen Pigmenten; besonders bevorzugt verwendet werden Ruß, Titandioxid, Eisenoxide, Chromoxide, Cobaltoxid und farbige Spinelle wie z.B. Kobaltaluminiumoxid.

Ein besonders hoher Glanz wird in der Regel bei Verwendung von Metallschichten erhalten, aber auch mit Metalloxidschichten können ästhetisch eindrucksvolle Glanzeffekte erzielt werden. Auch im Hinblick auf den erzielbaren Glanz können die erfindungsgemäßen Pigmente somit in einem weiten Bereich im Hinblick auf die jeweilige Anwendung optimiert werden.

Der ästhetische Gesamteindruck der erfindungsgemäßen Pigmente kann ggf. durch einen Interferenzeffekt unterstrichen und abgerundet werden, wie dies oben beschrieben worden ist. Bei den erfindungsgemäßen Pigmenten handelt es sich somit um optische Systeme mit einer hervorragenden Kombination optischer Eigenschaften, deren relative Ausprägung zudem im Hinblick auf die jeweilige Anwendung in einem weiten Bereich variiert und optimiert werden kann. Dabei kann diese Optimierung von einem Fachmann auf der Basis der vorliegenden Beschreibung routinemäßig durchgeführt werden, ohne daß es einer erfinderischen Tätigkeit bedürfte.

Die Herstellung der erfindungsgemäßen Pigmente erfolgt in einem kontinuierlichen Verfahren mit Hilfe eines endlosen Bandes, beispielsweise in einem kontinuierlichen Bandverfahren oder in einem kontinuierlichen Trommelverfahren.

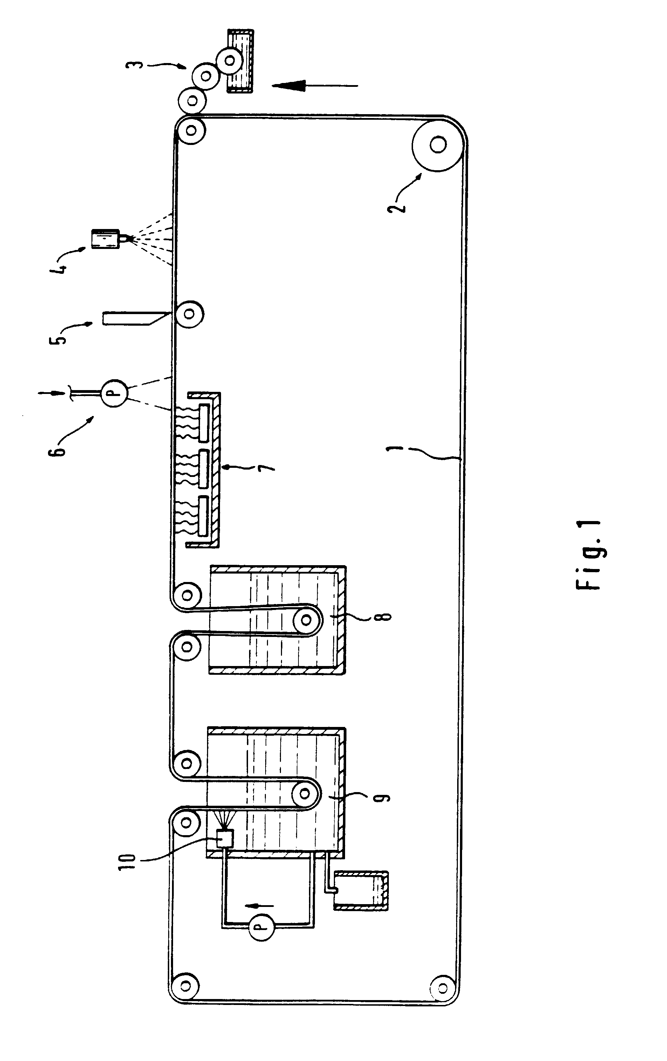

Zunächst soll an Hand der schematischen Skizze in Fig. 1 das Bandverfahren erläutert werden. Das endlose Band 1, welches über ein Rollensystem 2 geführt wird, durchläuft eine Aufgabestrecke, wo es mit einem dünnen Film des Precursors belegt wird. Das Auftragen des Precursors zusammen mit dem Farbmittel erfolgt nach bekannten Verfahren, beispielsweise über ein Walzensystem 3 oder eine Düse 4. Diese Düse kann als Einstoff- oder Mehrstoffdüse ausgestaltet sein. Zusätzlich kann zur Einstellung der Schichtdicke des aufgetragenen Films eine variable Blende 5 oder eine Luftbürste, bei der ein scharfer Luftstrahl durch eine Schlitzdüse geblasen wird, angeordnet sein. Eine Beschichtung des Bandes durch Rakeln (doctor blade) oder Tauchen (dip coating) ist ebenfalls möglich.

Als besonders vorteilhaft hat sich die Kombination von einseitiger oder beidseitiger Tauchbeschichtung des Bandes mit darauffolgender Nivellierung der aufgetragenen Schicht mit Hilfe einer Luftbürste erwiesen.

Das Farbmittel wird entweder vor dem Auftragen auf das Band im Precursor dispergiert oder gelöst, oder die Komponenten werden über mehrere Düsen getrennt auf das Band aufgetragen. Die Dispergierung der Pigmentpartikel im Precursor erfolgt nach bekannten Verfahren, beispielsweise durch Ultraschallbad. Die Dispergierung mit Hilfe einer Perlmühle hat sich als besonders vorteilhaft erwiesen.

Das beschichtete Band wird anschließend durch eine Trockenstrecke geführt, die aus einem oder mehreren Abschnitten bestehen kann. Eine bevorzugte Ausgestaltung der Trockenzone besteht aus einer Vortrocknungseinrichtung 6, in der der Film mit heißer Luft von 80-150 °C beaufschlagt wird, und einer nachfolgenden IR-Trocknungseinrichtung 7. Daneben sind aber auch weitere Ausgestaltungen der Trockenzone möglich. Die Gesamtheizleistung der Trockenzone ist u.a. abhängig von der Vorlaufgeschwindigkeit des Bandes und beträgt zwischen 0,5 und 10 kW pro m Bandbreite. Die Vorlaufgeschwindigkeit des Bandes liegt zwischen 1 und 1000 m/min und insbesondere zwischen 100 und 500 m/min, wobei aber auch größere Abweichungen von diesen Werten möglich sind. Der Fachmann kann die Heizleistung der Trockenzone und die Vorlaufgeschwindigkeit des Transportbandes ohne weiteres aneinander anpassen.

Die Verwendung von Wasserglas als Precursor erfordert ein Säurebad 8, in dem der auf das Band aufgetragene Alkalisilikatfilm zu Siliziumdioxid umgesetzt wird. Im Waschbehälter 9 werden anschließend die Alkaliionen aus der Matrix herausgewaschen.

Die Säurebehandlung erfolgt entweder durch Hindurchführen des Bandes durch einen mit Säure gefüllten Behälter oder durch Begasen des aufgetragenen Films mit Chlorwasserstoff.

Die Konzentration der Säure wird auf 1 % bis 20 %, vorzugsweise auf 5 % bis 15 % eingestellt. Als Säuren können verwendet werden: Salzsäure, Phosphorsäure, Schwefelsäure, Salpetersäure, Titantetrachloridlösung, Eisenchloridlösung, Zinnchloridlösung sowie organische Säuren, wie zum Beispiel Essigsäure.

Die Bedingungen im Säurebad müssen so gewählt werden, daß im wesentlichen nur die Alkali-Ionen herausdiffundieren, während die zugesetzten anderen Kationen zurückbleiben. Da Alkali-Ionen sehr hohe Diffusionskoeffizienten für die im Sauren erfolgende Herausdiffusion aus der SiO₂-Matrix aufweisen, kann ein selektives Herauslösen der Alkali-Ionen im allgemeinen erfolgen, indem die Verweilzeit des Films im Säurebad kurz gewählt wird oder indem eine schwache Säure wie z.B. Phosphorsäure benutzt wird.

Die gebildete Schicht wird anschließend durch eine Vorrichtung vom Band abgetrennt. Die Abtrennung kann entweder mechanisch durch Schaben oder Bürsten oder berührungslos durch Auflösen einer "release layer" oder durch Ultraschall erfolgen. Als vorteilhaft hat sich die Abtrennung mit einem Flüssigkeits- oder Gasstrahl 10 erwiesen. Besonders materialschonend ist die Ablösung des Films im feuchten Zustand, weil dieser dann weniger auf dem Band haftet und das feuchte Material das Band beim Ablösen weniger verkratzt.

Sollen die Matrixplättchen mit Metalloxidschichten belegt werden, wird dem Bandverfahren eine naßchemische Reaktionsschicht nachgeschaltet, bei dem das plättchenförmige Matrixmaterial in Wasser suspendiert wird und durch Zugabe einer oder mehrerer Metallsalzlösungen bei einem für die Abscheidung der jeweiligen Metalloxide oder Metallhyroxide geeigneten pH-Wert mit einer glatten Metalloxid bzw. -hydroxiddeckschicht überzogen werden. Es können auch Mischoxid- bzw. Hydroxidschichten und auch mehrere Deckschichten nacheinander abgeschieden werden. Dieser nachfolgende naßchemische Reaktionsschritt ist an sich bekannt und beschrieben in DE 19 59 988, DE 22 15 191, DE 22 44 298, DE 23 13 331, DE 25 22 572, DE 31 37 808, DE 31 37 809, DE 31 51 343, DE 31 51 355, DE 32 11 602 und DE 32 35 017.

Für eine bessere Haftung der Metalloxidschicht auf der Matrix ist es zweckmäßig, die Matrix zuerst mit einer Zinndioxidschicht zu belegen.

Sollen Pigmente mit einer Metalldeckschicht hergestellt werden, wird das Band mit dem getrockneten Film durch eine PVD-Zone geführt.

Die Beschichtung mit Metallen, beispielsweise mit Silber, Kupfer oder Gold, kann aber auch in einem naßchemischen Verfahren erfolgen.

Zur Gewährleistung eines kontinuierlichen Bandverfahrens wird das Band in diesem Falle vorzugsweise durch hintereinander geschaltete Vakuumkammern geführt, welche differentiell evakuiert werden. Die Metallabscheidung erfolgt dann in der Regel durch Aufdampfen, Aufsputtern oder Plasmapolymerisation in der innersten Kammer, welche das beste Vakuum aufweist.

Kontinuierliche Bandverfahren sind bekannt aus US 3 138 475 oder EP 0,240,952.

Anstelle der beschriebenen Anordnung können auch andere Vorrichtungen verwendet werden. So kann z.B. eine quasi-kontinuierliche Prozeßführung mit einer Anordnung realisiert werden, die sich an die in US 3,767,443, Fig. II widergegebene Vorrichtung anlehnt. Dabei ist ein sehr langes Trägerband auf eine Trommel aufgewickelt (Position 12 der Fig. II aus US 3,767,443); das Band wird dann über eine Belegungsstrecke abgewickelt, in der zunächst eine Release-layer und dann der Wasserglasfilm aufgebracht wird, der anschließend getrocknet und ggf. mit einer Mineralsäure umgesetzt wird. Dann wird das belegte Trägerband auf eine Speichertrommel (Position 15 der Fig. II) aufgewickelt. Nach Abwicklung des gesamten Trägerbandes wird die Speichertrommel in ein Bad gelegt, in dem sich die Release-layer auflöst, wobei sich der getrocknete Matrixfilm von dem Trägerband abtrennt. Im allgemeinen wird der belegte Trägerfilm vor dem Aufwickeln auf die Speichertrommel nicht mit einer Mineralsäure behandelt, da der erhaltene SiO₂-Film sich dabei bereits teilweise von dem Trägerband lösen kann. Nach der Auflösung der Release-layer liegen Matrixplättchen vor, die abgetrennt und mit einer Mineralsäure behandelt werden.

Die beschriebene Trommelanordnung kann auch für eine quasikontinuierliche Prozeßführung benutzt werden. Dazu wird das Trägerband in einem ersten Schritt mit dem Precursor belegt, der dann getrocknet wird. Nach vollständiger Aufwicklung des mit dem getrockneten Matrixfilm belegten Trägerbands auf die Speichertrommel wird der Precursorbehälter gegen ein Mineralsäurebad ausgetauscht; das belegte Trägerband wird dann durch das Mineralbad abgewickelt, wobei sich die Laufrichtung des Trägerbandes ändert und die Vorrats- und Speicherrollen vertauscht sind. Das Trägerband ist nach Passieren des Säurebads und ggf. einer Abstreifvorrichtung von dem Matrixfilm befreit, und ein neuer Zyklus kann beginnen. Dieses modifizierte Trommelverfahren ist eine Variante des in Fig. 1 gezeigten kontinuierlichen Bandverfahrens und hat gegenüber diesem häufig den Vorteil, daß die entsprechende Anordnung kompakter gebaut werden kann; nachteilig ist der nach der vollständigen Abwicklung des Bandes erforderliche Wechsel von Wasserglas- und Säurebehälter.

Die hier beschriebenen Anordnungen sind beispielhaft zu verstehen und sollen die Erfindung erläutern, ohne sie zu begrenzen. Allgemein sind kontinuierliche Prozeßführungen quasi-kontinuierlichen oder gar diskontinuierlichen Prozeßführungen gegenüber bevorzugt.

Es hat sich gezeigt, daß die Verwendung von Bändern, welche auf thermisch stabilen Kunststoffen basieren, vielfach vorteilhaft ist. Die Erweichungstemperatur des Kunststoffs sollte vorzugsweise nicht kleiner als 150 °C und insbesondere nicht kleiner als 180 °C sein, um ausreichend hohe Trocknungstemperaturen zu gewährleisten. Weiterhin sollte das Kunststoffmaterial chemisch weitgehend inert sein und insbesondere von verdünnten Mineralsäuren nicht angegriffen werden. Geeignete Bandmaterialien sind z.B. Polyethylenterephthalat, andere Polyester und Polyacrylate, wobei diese Aufzählung nur zur Erläuterung dient und die Erfindung nicht beschränken soll.

Die Kunststoffbänder weisen typischerweise eine Dicke von einigen 10 µm bis zu einigen mm auf, wobei i.a. Dicken zwischen 0,1 und 2 mm besonders bevorzugt sind. In Extremfällen können aber auch dickere Kunststoffbänder verwendet werden. Die Breite und Länge der Kunststoffbänder ist i.a. weniger kritisch und kann im Hinblick auf die jeweiligen Anforderungen optimiert werden.

Derartige Kunststoffbänder weisen in der Regel ohnehin eine glatte Oberfläche auf.

Es können aber auch dünne Metallbänder aus z.B. säureresistentem, beschichteten Edelstahl oder anderen gegenüber verdünnten Mineralsäuren inerten Metallen verwendet werden, wobei die geometrischen Abmessungen dieser Bänder im wesentlichen denen von Kunststoffbändern entsprechen. Die Metallbänder weisen eine hohe Flexibilität und Stabilität auf und können zur Erhöhung der Oberflächengüte nach herkömmlichen Verfahren poliert werden.

Auf das als Trägermedium verwendete Band kann ggf. zunächst eine Release-layer aufgebracht werden, um die Ablösung des Matrixfilms zu erleichtern; in quasi-kontinuierlichen Prozessen ist die Aufbringung einer Release-layer ggf. unumgänglich.

Als Release-layer kann z.B. eine dünne Schicht eines wasserlöslichen Polymers wie z.B. Polyvinylalkohol PVA dienen, die sich in dem Säurebad auflöst und dadurch zu einer vollständigen Abtrennng des Matrixfilms führt. In US 3 138 475 werden als Materialien für die Release-layer Silikonlacke und Stoffe wie z.B. Hartwachse, die sich beim Erhitzen ohne Rußbildung verflüchtigen oder zersetzen, vorgeschlagen. Die Verwendung einer Release-layer auch bei kontinuierlichen Prozessen hat den Vorteil, daß ggf. auf eine Abstreifvorrichtung verzichtet werden kann.

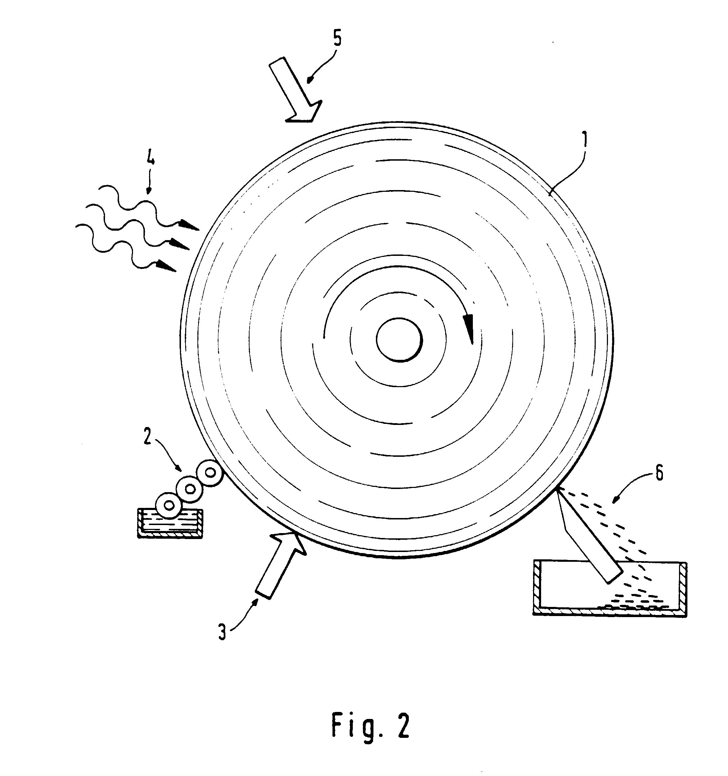

Die Herstellung der erfindungsgemäßen Pigmente kann auch mit Hilfe eines kontinuierlichen Trommelverfahrens erfolgen, das nachfolgend an Hand der schematischen Skizze in Figur 2 erläutert werden soll.

An einer rotierenden Trommel (1) sind in bestimmten Abschnitten die Vorrichtungen für die einzelnen Verfahrensstufen angeordent, wie sie bereits für ein endloses Band beschrieben wurden. Ein Precursor wird als dünner Film auf die säurebeständige Oberfläche aufgetragen. Das Auftragen erfolgt bevorzugt über ein Walzensystem 2, es kann aber auch durch eine Sprühvorrichtung 3 erfolgen. Die Trommel durchläuft dann eine Zone, die als Trockenzone oder Reaktionszone ausgelegt ist. Der flüssige Film wird hier verfestigt, indem er entweder über eine Vorrichtung 4 mit Strahlung beaufschlagt und/oder über eine Sprühvorrichtung 5 mit Reagenzien, zum Beispiel mit Säure, versetzt wird.

Die Verfestigung des Films kann durch Infrarotstrahlen, durch Mikrowellen, durch Heißluft oder durch Beheizen der Trommel von innen erfolgen.

Die erhaltene Schicht wird von der Trommeloberfläche durch eine Abtrennvorrichtung 6 abgetrennt. Das Abtrennen kann mit Hilfe einer Luftdüse, eines sogenannten Luftmessers, durch einen Wasserstrahl oder eine mechanische Vorrichtung erfolgen. Das erhaltene plättchenförmige Material wird dann, wie bereits beschrieben, weiterverarbeitet.

Als Precursor für die Herstellung der erfindungsgemäßen Pigmente wird vorzugsweise kommerziell erhältliches Wasserglas verwendet; so ist von E. Merck, Darmstadt unter der Bezeichnung Natronwasserglas reinst (Bestell-Nr. 5621) ein 35%iges Na-Wasserglas erhältlich, wobei es sich um auf die Masse des Wasserglases bezogene Massenprozente handelt.

Daneben können auch K- oder Ammonium-Wasserglas bzw. deren Mischungen verwendet werden. Das kommerziell erhältliche Wasserglas wird je nach seiner Konzentration vorzugsweise mit Wasser verdünnt, bis eine etwa 5-25%ige und insbesondere 10-20%ige wäßrige Lösung vorliegt.

Durch Zusätze können die Eigenschaften der Wasserglaslösung und der Matrix modifiziert werden. So bewirkt z.B. ein Zusatz von Na-Aluminatlösung (Si: Al-Verhältnis etwa 100:1) eine Änderung der Eigenschaften der Matrixteilchen, was zu einer besseren Verarbeitbarkeit führen kann. Besonders bevorzugte Zusätze sind Aluminat, Borat und/oder Phosphat.

Daneben können der Wasserglaslösung auch weitere Additive, beispielsweise oberflächenaktive Substanzen oder Viskositätserhöher zugesetzt werden.

Die Dicke des auf das Trägerband aufgebrachten Wasserglasfilms wird bei konstanter Bandgeschwindigkeit gegebenenfalls durch die variable Blende reguliert. Das Band wird in dem Blendenbereich durch geeignet angeordnete Rollen sehr planar gehalten, d.h. es wird jedes "Durchhängen" vermieden. Die Blende ist am unteren Ende vorzugsweise schneidenförmig ausgebildet; die Blendenkante wird sehr präzise justiert, wobei der Abstand zwischen Blendenunterkante und Bandoberfläche typischerweise zwischen etwa 1 und 20 µm beträgt. Der Abstand kann typischerweise auf eine Genauigkeit von etwa ± 1 µm oder weniger eingestellt werden. Da sich bei einer Wasserglaskonzentration von etwa 15 % die Dicke der nach dem Glühen erhaltenen SiO₂-Partikel etwa auf 1/10 der Dicke des ursprünglichen Wasserglasfilms vermindert hat, bedeutet dies, daß die Schichtdickentoleranz der SiO₂-Partikel etwa ± 0,1 µm beträgt; beträgt die Schichtdicke der SiO₂-Partikel 0,5 µm oder mehr, sind dies 20 % oder weniger, was i.a. als ausreichend betrachtet werden kann. Bei in der Praxis sehr gebräuchlichen Schichtdicken von etwa 1 µm oder mehr beträgt die Schichtdickentoleranz 10 % oder weniger. Sollen sehr dünne Plättchen mit einer Dicke von z.B. weniger als 0,5 µm mit einer Schichtdickentoleranz von etwa 10 % erhalten werden, können hierzu stärker verdünnte Wasserglaslösungen verwendet werden.

Durch die Wahl der Konzentration der Wasserglaslösung und der Trocknungsbedingungen kann die Schichtdicke und die Schichtdickentoleranz der erfindungsgemäßen Substrate gezielt beeinflußt werden, und es können auch sehr dünne Teilchen mit einer Schichtdickentoleranz (= Standardabweichung der Schichtdicke) von etwa 10 % erhalten werden. Plättchenförmige Siliciumsubstrate mit einer derart wohldefinierten Schichtdickenverteilung sind im Stand der Technik nicht beschrieben und mit den bekannten Herstellungsverfahren auch nicht zugänglich.

Die nach dem erfindungsgemäßen Verfahren hergestellten plättchenförmigen Pigmente zeichnen sich durch eine ausgezeichnete Oberflächengüte und eine sehr gleichmäßige Dicke aus. Die als Dickentoleranz bezeichnete Standardabweichung ist nicht größer als 10 %. Durch die planparallele Oberfläche und die enge Dickentoleranz der Matrixplättchen wird eine sehr große Farbreinheit und sehr hohe Farbstärke erreicht. Hinsichtlich ihrer Eigenschaften, z.B. ihres Deckvermögens, können sie im Hinblick auf die jeweilige Anwendung maßgeschneidert werden, da das Deckvermögen von der Anzahl der Pigmentpartikel in der Matrix abhängt. Je mehr Pigmentpartikel in der Matrix suspendiert werden, um so größer ist das Deckvermögen.

Die Farbe von Pigmenten, welche eingelagerte Rußpartikel enthalten, kann je nach der Konzentration der Rußpartikel z.B. von altweiß über hellgrau, metallfarbig, dunkelgrau bis hin zu schwarz reichen. Besonders interessant sind metallfarbige und insbesondere aluminiumfarbige Pigmente.

Die nach dem erfindungsgemäßen Verfahren hergestellten Pigmente haben ein sehr breites Anwendungsspektrum.

Sie können in Formulierungen wie Lacken, Kosmetika oder Kunststoffen verwendet werden. Die Pigmente können einen hohen Formfaktor (aspect ratio) aufweisen. Sie können daher Lacken oder Kunststoffen als Diffusionsbarriere, z.b. speziell als Korrosionsschutzmittel (Diffusionsbarriere für Sauerstoff) zugesetzt werden.

Die komplette Offenbarung aller vor- und nachstehend aufgeführten Anmeldungen, Patente und Veröffentlichungen sowie der korrespondierende Anmeldungen DE P 4134600 (18.10.1991), DE P 4138376 (22.11.1991), DE P 4212119 (10.04.1992), DE P 4215276 (09.05.1992) sind durch Bezugnahme in dieser Anmeldung enthalten.

Die im folgenden angegebenen Beispiele 1-14 und 16 beschreiben Ausgangsmateriale für die Herstellung der Matrix.

Die erfindungsgemäßen Plättchen werden im dem Beispielen 15 und 17-24 beschrieben.

### Beispiel 1

Ein umlaufendes Band aus Polyethylenterephthalat (Melinex O; Hersteller ICI; Breite: 12,5 cm; Geschwindigkeit: 10 m/min) wird durch Walzenbeschichtung im Gegenlauf (revers roll coating) mit einem Wasserglasfilm von etwa 10 µm Dicke belegt. Die Wasserglaslösung wird durch Verdünnen von handelsüblichem Wasserglas (Natronwasserglas reinst, Hersteller: E. Merck) mit Wasser im Verhältnis 1 zu 2,5 hergestellt, wobei noch 0,2 Gew.% eines Tensides (Pricol extra, Hersteller: Henkel) zugemischt werden. Die verdünnte Wasserglaslösung hat einen Siliziumdioxidgehalt von 7,7 %. Der Wasserglasfilm wird in einer Trocknungsstrecke durch Beaufschlagung mit Infrarotstrahlung und Heißluft getrocknet. Anschließend durchläuft das Band ein Säurebad mit 10%iger Salzsäure und nachfolgend ein Wasserbad. Beim Verlassen des Wasserbades wird der gelartige Siliziumdioxidfilm durch einen Wasserstrahl abgelöst und in das Wasserbad gespült. Die plättchenförmigen Bruchstücke werden in einer Filterschleuder filtriert und mit vollentsalztem Wasser gewaschen, bis sich ein pH-Wert von 5 bis 6 einstellt. Der Filterkuchen wird in einer Labormühle (Rotor GT 800, Hersteller: Rotor) in wäßriger Suspension bei Stufe 5 zerkleinert.

Die erhaltenen SiO₂-Plättchen haben einen Durchmesser von 20 bis 100 µm und eine durchschnittliche Dicke von 500 nm.

### Beispiel 2

6 g Benetzungshilfsmittel (Pricol GV; Hersteller: Henkel) werden in 2 1 vollentsalztem Wasser gelöst. In diese Lösung wird 1 1 Natronwasserglas reinst (Hersteller: E. Merck) eingerührt, Zu dieser Lösung werden 100 ml einer Aluminat-Stammlösung gegeben, die durch Lösen von 32 g Natriumaluminat in 500 ml vollentsalztem Wasser hergestellt wird.

Die Lösung wird, wie in Beispiel 1 beschrieben, auf einem endlosen Band verarbeitet.

Man erhält eine plättchenförmige SiO₂-Matrix mit Aluminium als Netzwerkbildner in einer Konzentration von 1 Atom-% bezogen auf Silizium.

### Beispiel 3

6 g Benetzungshilfsmittel (Pricol GV; Hersteller: Henkel) werden in 2 1 vollentsalztem Wasser gelöst. In diese Lösung wird 1 1 Natronwasserglas reinst (Hersteller: E. Merck) eingerührt. Zu dieser Lösung werden 100 ml einer Borat-Stammlösung gegeben, die durch Lösen von 21 g Borsäure (Hersteller: E. Merck) in 300 ml vollentsalztem Wasser und Einstellen des pH-Wertes mit verdünnter Natronlauge auf einen Wert von 12 hergestellt wird.

Die Lösung wird, wie in Beispiel 1 beschrieben, auf einem endlosen Band verarbeitet.

Man erhält eine plättchenförmige SiO₂-Matrix mit Bor als Netzwerkbildner in einer Konzentration von 1 Atom-% bezogen auf Silizium.

### Beispiel 4

6 g Benetzungshilfsmittel (Pricol GV; Hersteller: Henkel) werden in 2 1 vollentsalztem Wasser gelöst. In diese Lösung wird 1 1 Natronwasserglas reinst (Hersteller: E. Merck) eingerührt. Zu dieser Lösung werden 100 ml einer Phosphat-Stammlösung gegeben, die durch Lösen von 24 g Na₂HPO₄ × 12 H₂O (Hersteller: E. Merck) in 300 ml vollentsalztem Wasser und Einstellen des pH-Wertes mit verdünnter Natronlauge auf einen Wert von 12 hergestellt wird.

Die Lösung wird, wie in Beispiel 1 beschrieben, auf einem endlosen Band verarbeitet.

Man erhält eine plättchenförmige SiO₂-Matrix mit Phosphor als Netzwerkbildner in einer Konzentration von 1 Atom-% bezogen auf Silizium.

### Beispiel 5

100 g SiO₂-Plättchen werden in 1500 ml vollentsalztem Wasser suspendiert. Zunächst wird unter starkem Rühren bei 75 °C und pH 1,8 eine SnCl₄-Lösung (Herstellung: 11,5 g SnCl₄ werden mit 38 ml konzentrierter Salzsäure versetzt und diese Lösung mit vollentsalztem Wasser auf 192 ml aufgefüllt) mit einer Dosierrate von 0,5 ml/min zugetropft. Anschließend wird unter gleichen Bedingungen und mit gleicher Dosierrate eine TiCl₄-Lösung (TiCl₄-Gehalt: 375 g/l) zugetropft. Der pH-Wert wird jeweils durch Zugabe von 16%iger Natronlauge konstant gehalten.

Nach Zugabe von etwa 110 ml wird das Produkt filtriert, mit vollentsalztem Wasser neutral gewaschen, getrocknet und bei 800 °C 30 min geglüht.

Man erhält ein hoch transparentes, silberfarbenes Interferenzpigment mit ausgezeichnetem Glanz.

Die coloristischen Daten im Vergleich mit einem silberfarbenen Interferenzpigment auf Basis von Glimmer (Iriodin 130, Hersteller: E. Merck) sind in Tabelle 1 enthalten.

### Beispiel 6

100 g SiO₂-Plättchen werden in 1500 ml vollentsalztem Wasser suspendiert. Zunächst wird unter starkem Rühren bei 75 °C und pH 1,8 eine SnCl₄-Lösung (Herstellung: 11,5 g SnCl₄ werden mit 38 ml konzentrierter Salzsäure versetzt und diese Lösung mit vollentsalztem Wasser auf 192 ml aufgefüllt) mit einer Dosierrate von 0,5 ml/min zugetropft. Anschließend wird unter gleichen Bedingungen und mit gleicher Dosierrate eine TiCl₄-Lösung (TiCl₄-Gehalt: 375 g/l) zugetropft. Der pH-Wert wird jeweils durch Zugabe von 16%iger Natronlauge konstant gehalten.

Nach Zugabe von etwa 220 ml wird das Produkt filtriert, mit vollentsalztem Wasser neutral gewaschen, getrocknet und bei 800 °C 30 min geglüht.

Man erhält ein hoch transparentes, goldgelbes Inteferenzpigment mit ausgezeichnetem Glanz.

Die coloristischen Daten im Vergleich mit einem goldgelben Interferenzpigment auf Basis von Glimmer (Iriodin 207, Hersteller: E. Merck) sind in Tabelle 1 enthalten.

### Beispiel 7

100 g SiO₂-Plättchen werden in 1500 ml vollentsalztem Wasser suspendiert. Zunächst wird unter starkem Rühren bei 75 °C und pH 1,8 eine SnCl₄-Lösung (Herstellung: 11,5 g SnCl₄ werden mit 38 ml konzentrierter Salzsäure versetzt und diese Lösung mit vollentsalztem Wasser auf 192 ml aufgefüllt) mit einer Dosierrate von 0,5 ml/min zugetropft. Anschließend wird unter gleichen Bedingungen und mit gleicher Dosierrate eine TiCl₄-Lösung (TiCl₄-Gehalt: 375 g/l) zugetropft. Der pH-Wert wird jeweils durch Zugabe von 16%iger Natronlauge konstant gehalt ten. Gegebenenfalls müssen durch Nebenhydrolyse entstehende Produkte durch Sedimentation beseitigt werden.

Nach Zugabe von etwa 570 ml wird das Produkt filtriert, mit vollentsalztem Wasser neutral gewaschen, getrocknet und bei 800 °C 30 min geglüht.

Man erhält ein hoch transparentes, rotes Interferenzpigment mit ausgezeichnetem Glanz.

Die coloristischen Daten im Vergleich mit einem roten Interferenzpigment auf Basis von Glimmer (Iriodin 215, Hersteller: E. Merck) sind in Tabelle 1 enthalten.

### Beispiel 8

100 g SiO₂-Plättchen werden in 1500 ml vollentsalztem Wasser suspendiert. Unter starkem Rühren wird bei 75 °C und pH 4,0 eine FeCl₃-Lösung (FeCl₃-Gehalt: 5,4 %) mit einer Geschwindigkeit von 0,5 ml/min zugetropft. Der pH-Wert wird durch Zugabe von verdünnter Natronlauge konstant gehalten.

Nach Zugabe von etwa 1200 ml wird das Produkt filtriert, mit vollentsalztem Wasser neutral gewaschen, getrocknet und bei 800 °C 30 min geglüht.

Man erhält ein leuchtend bronzefarbenes Effektpigment mit ausgezeichnetem Glanz und hoher Brillanz.

Die coloristischen Daten im Vergleich mit einem bronzefarbenen Effektpigment auf Basis von Glimmer (Iriodin 530, Hersteller: E. Merck) sind in Tabelle 1 enthalten.

### Beispiel 9

100 g SiO₂-Plättchen werden in 1500 ml vollentsalztem Wasser suspendiert. Unter starkem Rühren wird bei 75 °C und pH 4,0 eine Eisenchlorid-Lösung (FeCl₃-Gehalt: 5,4 %) mit einer Geschwindigkeit von 0,5 ml/min zugetropft. Der pH-Wert wird durch Zugabe von verdünnter Natronlauge konstant gehalten. Gegebenenfalls müssen durch Nebenhydrolyse entstehende Produkte durch Sedimentation beseitigt werden.

Nach Zugabe von etwa 1375 ml wird das Produkt filtriert, mit vollentsalztem Wasser neutral gewaschen, getrocknet und bei 800 °C 30 min geglüht.

Man erhält ein hoch transparentes, rotes Effektpigment mit ausgezeichnetem Glanz.

Die coloristischen Daten im Vergleich mit einem weinroten Effektpigment auf Basis von Glimmer (Iriodin 534, Hersteller: E. Merck) sind in Tabelle 1 enthalten.

### Beispiel 10

Zu der in Beispiel 1 beschriebenen Wasserglaslösung werden außer 0,2 Gew.% eines Tensides (Pricol extra, Hersteller: Henkel) zusätzlich 0,2 Gew.% Methylenblau (Hersteller: E. Merck) zugegeben. Das Gemisch wird wie im Beispiel 1 beschrieben, auf ein endloses Band aufgebracht und verarbeitet. Das nach dem Mahlen in der Labormühle erhaltene Produkt wird 60 Minuten bei 110 °C getrocknet.

Man erhält blaue, glänzende SiO₂-Plättchen mit hoher Transparenz.

### Beispiel 11

200 g der gemäß Beispiel 7 erhaltenen blauen SiO₂-Plättchen werden in 2 l vollentsalztem Wasser suspendiert, die Suspension auf 60 °C erwärmt und mit 2 n Natronlauge auf pH 9 eingestellt. Unter starkem Rühren und Konstanthalten der Temperatur werden 250 ml Natronwasserglaslösung mit einer Geschwindigkeit von 1 ml/min zudosiert. Die Natronwasserglaslösung wird hergestellt, indem 26 ml Natronwasserglas reinst (Hersteller: E. Merck) mit 224 ml vollentsalztem Wasser verdünnt werden. Durch kontrollierte Zugabe von verdünnter Salzsäure wird bei pH-Wert bei 9 gehalten.

Nach der Beschichtung mit SiO₂ wird das Produkt direkt mit einer reflektierenden Metalloxid-Interferenzschicht nach bekannten Verfahren, beispielsweise nach dem in US 4 086 100, Beispiel 1 beschriebenen Verfahren, weiterbeschichtet.

### Beispiel 12

Zu der in Beispiel 1 beschriebenen Wasserglaslösung wird eine wäßrige Lösung von Zitronensäure (Hersteller: E. Merck) und CrCl₃ × 6 H₂O (Hersteller: E. Merck) gegeben, um eine Konzentration von 7 % Zitronensäure und 3 % CrCl₃ × 6 H₂O in der Wasserglaslösung zu erhalten. Außerdem werden 3 % Texapon® (Hersteller: Henkel) als Benetzhilfsstoff zugegeben. Das Gemisch wird, wie in Beispiel 1 beschrieben, auf ein endloses Band aufgebracht und verarbeitet. Das nach dem Mahlen in der Labormühle erhaltene Produkt wird 60 min bei 110 °C getrocknet. Man erhält glänzende, olivgrüne Plättchen mit hoher Transparenz.

### Beispiel 13

Zu der in Beispiel 1 beschriebenen Wasserglaslösung wird eine wäßrige Lösung von Natriummethylendiamintetraacetat (Hersteller: E. Merck) und CoCl₂ × 6 H₂O (Hersteller: E. Merck) gegeben, um eine Konzentration von 8 % Natriummethylendiamintetraacetat und 3 % CoCl₂ × 6 H₂O in der Wasserglaslösung zu erhalten. Außerdem werden 3 % Texapon® (Hersteller: Henkel) als Benetzhilfsstoff zugegeben. Das Gemisch wird, wie in Beispiel 1 beschrieben, auf ein endloses Band aufgebracht und verarbeitet. Das nach dem Mahlen in der Labormühle erhaltene Produkt wird 60 min bei 110 °C getrocknet. Man erhält glänzende, lilafarbene Plättchen mit hoher Transparenz.

### Beispiel 14

Zu der in Beispiel 1 beschriebenen Wasserglaslösung wird eine wäßrige Lösung von Weinsäure (Hersteller: E. Merck) und FeCl₃ × 6 H₂O (Hersteller: E. Merck) gegeben, um eine Konzentration von 6 % Weinsäure und 8 % FeCl₃ × 6 H₂O in der Wasserglaslösung zu erhalten. Außerdem werden 3 % Texapon® (Hersteller: Henkel) als Benetzhilfsstoff zugegeben. Das Gemisch wird, wie in Beispiel 1 beschrieben, auf ein endloses Band aufgebracht und verarbeitet. Das nach dem Mahlen in der Labormühle erhaltene Produkt wird 60 min bei 110 °C getrocknet. Man erhält glänzende, bräunlich-gelbe Plättchen mit hoher Transparenz.

### Beispiel 15

In einer Perlmühle (Dispermat CV; Hersteller: VMA-Getzmann) werden 500 ml einer 3%igen Dispersion von Titandioxid-Partikeln (R506; Hersteller: Sachtleben Chemie; durchschnittliche Teilchengröße: 440 nm) in einer Wasserglaslösung (Natronwasserglas reinst; Hersteller: E. Merck; Verdünnungsverhältnis mit Wasser 1:2,5) hergestellt. Zur besseren Dispergierbarkeit werden 0,5 Gew.%, bezogen auf Titandioxid eines Stabilisators (HYDROPALAT® 884, Hersteller: Henkel) sowie zur besseren Benetzbarkeit des endlosen Bandes 1 Gew.%, bezogen auf die fertige Dispersion eines Benetzhilfsmittels (HYDROPALAT® 875, Hersteller: Henkel) zugesetzt.

Die Dispersion wurde, wie in Beispiel 1 beschrieben, auf ein endloses Band als dünner Film aufgebracht und das Band durch eine Trocknungsstrecke geführt, wo der Film durch Infrarotstrahlen und Heißluft getrocknet wird. Anschließend wird das Band durch ein Säurebad und ein Wasserbad geführt. Beim Verlassen des Wasserbades wird der gelartige Film durch einen Wasserstrahl vom Band abgelöst und in das Wasserbad gespült. Die abgelösten Bruchstücke des Film werden wie in Beispiel 1 weiterverarbeitet.

100 g der aus der Labormühle erhaltenen Plättchen werden in 2,5 1 vollentsalztem Wasser suspendiert und nach dem in US 4 086 100, Beispiel 1 beschriebenen Verfahren mit Titandioxid in der Rutilform belegt.

Man erhält ein hochdeckendes, weißes Pigment mit einem silbernen Glanz.

### Beispiel 16

| | |
|---|---|
| 12,3 g | DPP-Rot (Hersteller: Ciba Geigy), Teilchengröße: 50 nm, |
| 1,2 g | Stabilisator W-22 (Hersteller: Krahn-Chemie), |
| 50 g | vollentsalztes Wasser und |
| 200 g | Zirkonperlen (1-2,5 mm) |

werden für 90 min bei 2000 U/min gemahlen. Anschließend werden die Perlen gesiebt und die erhaltene Suspension mit 800 ml vollentsalztem Wasser sowie 300 ml Natronwasserglas reinst (Hersteller: E. Merck) verrührt. Das Gemisch wird, wie in Beispiel 1 beschrieben, auf einem endlosen Band verarbeitet. Die aus der Labormühle erhaltenen Plättchen werden bei 110 °C für eine Stunde getrocknet. Man erhält ein glänzendes, rotes Pigment.

### Beispiel 17

50 g der in Beispiel 16 erhaltenen Plättchen werden in 1 1 Wasser suspendiert. Zunächst wird unter kräftigem Rühren bei 75 °C und pH 1,8 eine SnCl₄-Lösung (Herstellung: 5,8 g SnCl₄ · 5 H₂O in 7 ml konzentrierter Salzsäure sowie 75 ml Wasser gelöst) mit einer Rate von 0,6 ml/min zudosiert. Anschließend wird die Temperatur auf 90 °C erhöht und der pH auf 1,5 abgesenkt. Während der Zugabe von TiCl₄-Lösung (TiCl₄-Gehalt = 380 g/l) mit 0,6 ml/min wird der pH jeweils durch Zugabe von verdünnter Natronlauge konstant gehalten.

Nach Zugabe von etwa 100 ml TiCl₄-Lösung erhält man eine silberfarbene Interferenz, nach 195 ml eine rote, nach 275 ml eine blaue und nach 300 ml eine grüne.

Die Produkte werden filtriert, gewaschen und getrocknet. Die verschiedenen Interferenzfarbpigmente zeigen blickwinkelunabhängig die rote Körperfarbe des Substrates und blickwinkelabhängig die Interferenzfarbe durch die TiO₂-Beschichtung, d.h. einen Farbflopp-Effekt.

### Koloristische Meßwerte zum Farbflopp-Pigment

Die angegebenen Meßwerte sind CIE-L*A*B* Werte einer 1,7 % Lackkarte auf schwarzem Untergrund. Die Meßgeometgrie betrug: die Interferenzfarbe 70° (Beleuchten) / 95° (Messen) [Glanz], die Körperfarbe 45° (Beleuchten) / 90° (Messen).

| **Interferenzfarbe** | | **A*** | **B*** | | **A*** | **B*** |
|---|---|---|---|---|---|---|
| **silber** | 70°/95° | 2,8 | -1, 9 | 45°/90° | 14,7 | 0,8 |
| | Glanz | silber | | nach Körperfarbe | rot | |
| **rot** | 70°/95° | 25,3 | 6,4 | 45°/90° | 21,2 | 8,1 |
| | Glanz | rot | | nach Körperfarbe | rot | |
| **blau** | 70°/95° | -12,7 | -11,5 | 45°/90° | 6,4 | -3,3 |
| | Glanz | blau | | nach Körperfarbe | rot | |
| **grün** | 70°/95° | -13,0 | 13,9 | 45°/90° | 6,4 | 3,1 |
| | Glanz | grün | | nach Körperfarbe | rot | |

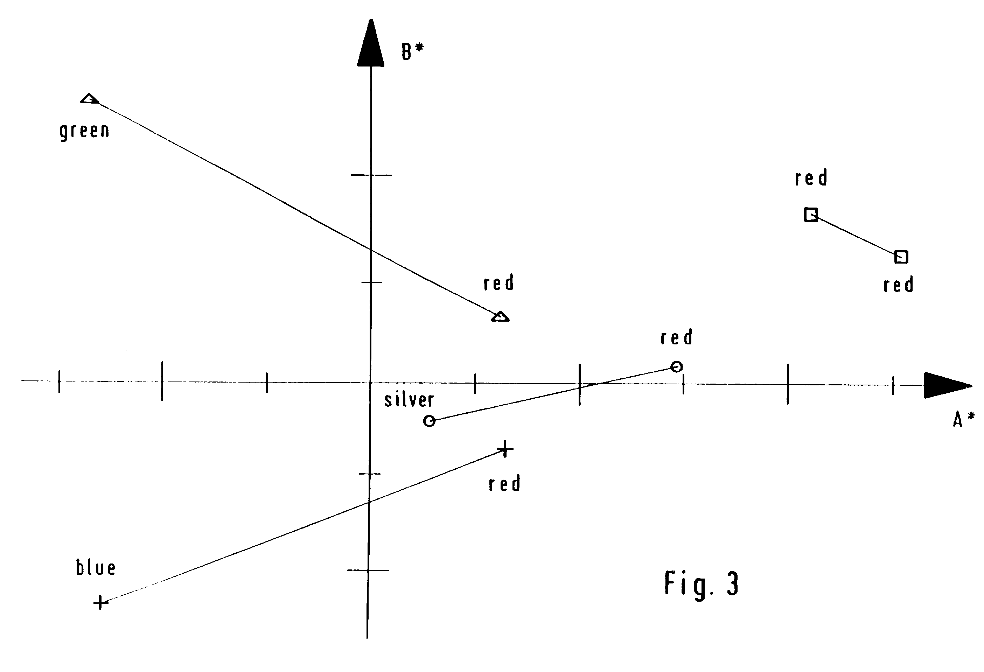

Die erhaltenen Farborte sind in Fig. 3 dargestellt.

### Beispiel 18

82,2 g DERUSSOL C (Rußdispersion w = 21 %, Hersteller: Degussa) werden mit 300 ml Natronwasserglas reinst (Hersteller: E. Merck) und 680 ml vollentsalztem Wasser verrührt. Anschließend werden 2,1 g Pricol (Hersteller: Henkel) als Benetzhilfsmittel in der Suspension aufgelöst. Die Suspension wird, wie in Beispiel 1 beschrieben, auf einem endlosen Band verarbeitet. Die aus der Labormühle erhaltenen Plättchen werden in Wasser suspendiert und mit Titandioxid nach dem in US 4 086 100 beschriebenen Verfahren beschichtet.

Die beschichteten Plättchen werden bei 800 °C unter Schutzgas geglüht und in bekannter Weise aufgearbeitet. Man erhält in Abhängigkeit von der Dicke der TiO₂-Interferenzschicht verschiedenfarbige (z.B. silber, gold, rot, blau, grün) Körperfarbenpigmente.

### Beispiel 19

| | |
|---|---|
| 115,3 g | Eisenoxid-rot (Hersteller: BAYER), |
| 3,9 g | Stabilisator W-22 (Hersteller: Krahn-Chemie), |
| 600 g | vollentsalztes Wasser und |
| 200 g | Zirkonperlen (Durchmesser: 1-2,5 mm) |

werden für 1 Stunde bei 3000 U/min in einer Perlmühle gemahlen. Nach dem Absieben der Perlen wird die Suspension mit 2250 ml vollentsalztem Wasser und 1140 ml Natronwasserglas reinst (Hersteller: E. Merck) verrührt.

Die Suspension wird, wie in Beispiel 1 beschrieben, auf einem endlosen Band verarbeitet. Die aus der Labormühle erhaltenen, roten Plättchen werden in Wasser suspendiert und mit Metalloxiden nach bekannten Verfahren, beispielsweise nach dem in US 4 086 100 beschriebenen Verfahren, auf Interferenzfarben beschichtet.

### Beispiel 20

| | |
|---|---|
| 115,3 g | Eisenoxid-gelb (Hersteller: BAYER), |
| 3,9 g | Stabilisator W-22 (Hersteller: Krahn-Chemie), |
| 600 g | vollentsalztes Wasser und |
| 200 g | Zirkonperlen (Durchmesser: 1-2,5 mn) |

werden für 1 Stunde bei 3000 U/min in einer Perlmühle gemahlen. Nach dem Absieben der Perlen wird die Suspension mit 2250 ml vollentsalztem Wasser und 1140 ml Natronwasserglas reinst (Hersteller: E. Merck) vermischt.

Die Suspension wird, wie in Beispiel 1 beschrieben, auf einem endlosen Band verarbeitet. Die aus der Labormühle erhaltenen, gelben Plättchen werden in Wasser suspendiert und mit Metalloxiden nach bekannten Verfahren, beispielsweise nach dem in US 4 086 100 beschriebenen Verfahren, auf Interferenzfarben beschichtet.

### Beispiel 21

| | |
|---|---|
| 115,3 g | Eisenoxid-schwarz (Hersteller: CRODA) |
| 3,9 g | Stabilisator W-22 (Hersteller: Krahn-Chemie), |
| 600 g | vollentsalztes Wasser und |
| 200 g | Zirkonperlen (Durchmesser: 1-2,5 mm) |

werden für 1 Stunde bei 3000 U/min in einer Perlmühle gemahlen. Nach dem Absieben der Perlen wird die Suspension mit 2250 ml vollentsalztem Wasser und 1140 ml Natronwasserglas reinst (Hersteller: E. Merck) vermischt.

Die Suspension wird, wie in Beispiel 1 beschrieben, auf einem endlosen Band verarbeitet. Die aus der Labormühle erhaltenen, schwarzen Plättchen werden in Wasser suspendiert und mit Metalloxiden nach bekannten Verfahren, beispielsweise nach dem in US 4 086 100 beschriebenen Verfahren, auf Interferenzfarben beschichtet.

### Beispiel 22

| | |
|---|---|
| 115,3 g | Chromoxid (Hersteller: CRODA) |
| 3,9 g | Stabilisator W-22 (Hersteller: Krahn-Chemie), |
| 600 g | vollentsalztes Wasser und |
| 200 g | Zirkonperlen (Durchmesser: 1-2,5 mm) |

werden für 1 Stunde bei 3000 U/min in einer Perlmühle gemahlen. Nach dem Absieben der Perlen wird die Suspension mit 2250 ml vollentsalztem Wasser und 1140 ml Natronwasserglas reinst (Hersteller: E. Merck) vermischt.

Die Suspension wird, wie in Beispiel 1 beschrieben, auf einem endlosen Band verarbeitet. Die aus der Labormühle erhaltenen, grünen Plättchen werden in Wasser suspendiert und mit Metalloxiden nach bekannten Verfahren, beispielsweise nach dem in US 4 086 100 beschriebenen Verfahren, auf Interferenzfarben beschichtet.

### Beispiel 23

| | |
|---|---|
| 115,3 g | Berliner Blau (Hersteller: CRODA) |
| 3,9 g | Stabilisator W-22 (Hersteller: Krahn-Chemie), |
| 600 g | vollentsalztes Wasser und |
| 200 g | Zirkonperlen (Durchmesser: 1-2,5 mm) |

werden für 1 Stunde bei 3000 U/min in einer Perlmühle gemahlen. Nach dem Absieben der Perlen wird die Suspension mit 2250 ml vollentsalztem Wasser und 1140 ml Natronwasserglas reinst (Hersteller: E. Merck) vermischt.

Die Suspension wird, wie in Beispiel 1 beschrieben, auf einem endlosen Band verarbeitet. Die aus der Labormühle erhaltenen, blauen Plättchen werden in Wasser suspendiert und mit Metalloxiden nach bekannten Verfahren, beispielsweise nach dem in US 4 086 100 beschriebenen Verfahren, auf Interferenzfarben beschichtet.

### Beispiel 24

100 g der gemäß Beispiel 1 erhaltenen SiO₂-Plättchen werden in einem Gemisch aus

| | |
|---|---|
| 1000 ml | einer 3%igen Silbernitratlösung, |
| 25 ml | einer 40%igen Formalinlösung und |
| 25 ml | Methanol |

dispergiert. Die Suspension wird über Nacht langsam gerührt, bis alles Silber aus der Lösung auf die SiO₂-Plättchen abgeschieden worden ist.

Die erhaltenen silbergrauen Plättchen werden gewaschen, getrocknet und bei 800 °C geglüht.

Man erhält ein silbergraues Pigment.

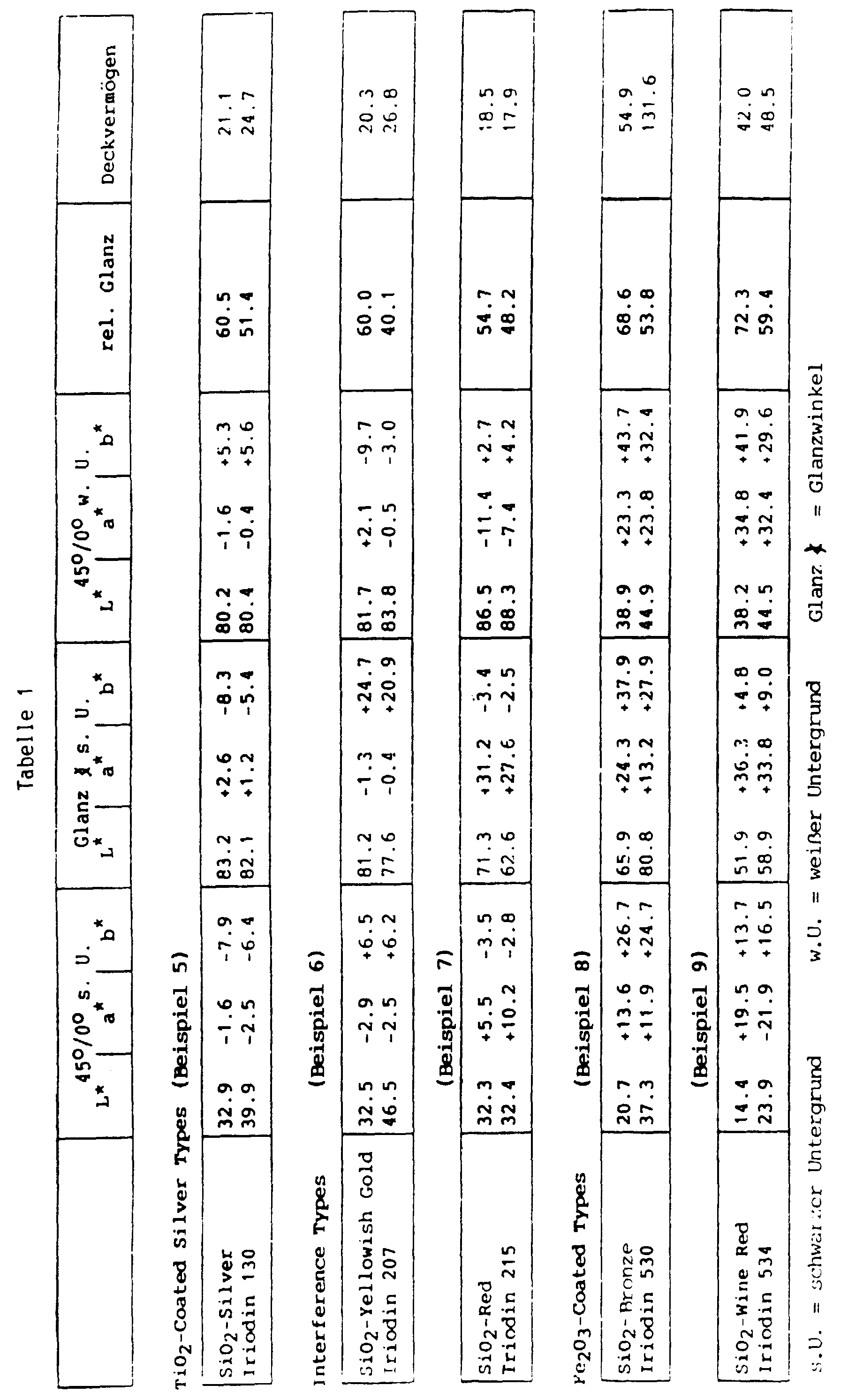

## Patentansprüche

1. Plättchenförmiges Pigment mit hohem Glanz und hohem Deckvermögen oder hoher Transparenz, bestehend aus einer durch Verfestigung eines flüssigen Precursors hergestellten transparenten, anorganischen, plättchenförmigen Matrix, die zur Erzielung des Glanzes mit einer oder mehreren dünnen, transparenten oder semitransparenten reflektierenden Schichten aus Metalloxiden oder Metallen belegt ist, dadurch erhältlich, daß
- der Precursor des Matrixmaterials als dünner Film auf ein endloses Band aufgebracht wird,
- der flüssige Film durch Trocknung verfestigt wird,
- im verfestigten Film die Matrix durch eine chemische Reaktion aus dem Precursor entwickelt wird,
- der durch Trocknung verfestigte Film anschließend mit einer Säure behandelt wird,
- der Film anschließend vom Trägermedium getrennt und gewaschen wird,
- die Partikel gegebenenfalls getrocknet, geglüht, gemahlen und klassiert werden und
- die erhaltenen Filmpartikel mit einer oder mehreren Schichten aus Metalloxiden oder Metallen belegt werden.

2. Pigment nach Anspruch 1, dadurch gekennzeichnet, daß die Matrix als zusätzlichen Bestandteil ein lösliches oder unlösliches Farbmittel enthält.

3. Pigment nach Ansprüchen 1 und 2, dadurch gekennzeichnet, daß die transparente anorganische Matrix aus Siliciumdioxid, Silikat, Boroxid, Borat, Aluminiumoxid, Aluminat oder Gemischen derselben besteht.

4. Pigment nach den Ansprüchen 1 bis 3, dadurch gekennzeichnet, daß die anorganische Matrix mit Netzwerkbildnern bzw. Netzwerkwandlern modifiziert ist.

5. Pigment nach den Ansprüchen 1 bis 4, dadurch gekennzeichnet, daß die anorganische Matrix zur Glättung der Oberfläche Bariumsulfat enthält.

6. Pigment nach Anspruch 5, dadurch gekennzeichnet, daß das Bariumsulfat in einer Menge von 1 Gew.-% bis 50 Gew.-%, vorzugsweise 10 Gew.-% bis 25 Gew.-%, im unbeschichteten Substrat enthalten ist.

7. Pigment nach den Ansprüchen 1 bis 6, dadurch gekennzeichnet, daß das nichtlösliche Farbmittel ein organisches oder anorganisches Pigment ist.

8. Pigment nach Anspruch 7, dadurch gekennzeichnet, daß das organische Pigment ein Azopigment, ein Anthrachinon-Pigment, ein Indigo- oder Thioindigo-Derivat, eine Diketo-Pyrrolo-Pyrrol-Pigment, ein Perylen-Pigment oder ein Phtha-locyanin-Pigment ist.

9. Pigment nach Anspruch 7, dadurch gekennzeichnet, daß das anorganische Pigment Ruß, Titandioxid, Eisenoxid, Chromdioxid, Cobaltoxid oder ein Mischoxid ist.

10. Pigment nach den Ansprüchen 1 bis 6, dadurch gekennzeichnet, daß das lösliche Farbmittel eine lösliche Metallverbindung, die zu einem farbgebenden Metalloxid führt, oder ein lösliches organisches Pigment ist.

11. Pigment nach Anspruch 10, dadurch gekennzeichnet, daß das farbgebende Metalloxid Eisenoxid, Chromoxid oder Kobaltoxid ist.

12. Pigment nach Anspruch 10, dadurch gekennzeichnet, daß das lösliche organische Pigment ein in Lauge löslicher Hydroxyanthrachinonfarbstoff oder ein saurer Azofarbstoff ist.

13. Pigment nach den Ansprüchen 1 bis 12, dadurch gekennzeichnet, daß die als reflektierende Schicht ausgebildete Metallschicht aus Silber, Gold, Palladium, Platin, Aluminium, Chrom oder Kupfer besteht.

14. Pigment nach den Ansprüchen 1 bis 12, dadurch gekennzeichnet, daß die als reflektierende Schicht ausgebildete Metalloxidschicht aus Chromoxid, Titandioxid, Eisenoxid oder einem Gemisch derselben besteht und zur Erzielung von Interferenzfarben dient.

15. Pigment nach den Ansprüchen 1 bis 14, dadurch gekennzeichnet, daß auf die reflektierende Schicht oder die Matrix weitere Schichten für spezielle physikalische und/oder chemische Anforderungen aufgebracht sind.

16. Verfahren zur Herstellung eines plättchenförmigen Pigmentes mit hohem Glanz und hohem Deckvermögen oder hoher Transparenz, bestehend aus einer durch Verfestigung eines flüssigen Precursors hergestellten transparenten, anorganischen, plättchenförmigen Matrix, die mit einer oder mehreren dünnen, transparenten oder semitransparenten reflek-tierenden Schichten aus Metalloxiden oder Metallen belegt ist, wobei
- der Precursor des Matrixmaterials als dünner Film auf ein endloses Band aufgebracht wird,
- der flüssige Film durch Trocknung verfestigt wird,
- im verfestigten Film die Matrix durch eine chemische Reaktion aus dem Precursor entwickelt wird,
- die entstandene Schicht anschließend vom Trägermedium getrennt und gewaschen wird und
- die Partikel gegebenenfalls getrocknet, geglüht, gemahlen und klassiert werden,
dadurch gekennzeichnet, daß der durch Trocknung verfestigte Film anschließend mit einer Säure behandelt wird und die erhaltenen Filmpartikel mit einer oder mehreren reflektierenden Schichten aus Metalloxiden oder Metallen belegt werden.

17. Verfahren nach Anspruch 16, dadurch gekennzeichnet, daß als Precursor Lösungen von anorganischen oder organischen Verbindungen der Metalle Aluminium, Silicium, Kalium oder Natrium mit Boraten, Chloride, Aluminaten, Poly- bzw. Metaphosphaten, Silikaten oder Gemischen derselben eingesetzt werden.

18. Verfahren nach den Ansprüchen 16 bis 17, dadurch gekennzeichnet, daß der Precursor Wasserglas ist.

19. Verfahren nach den Ansprüchen 16 bis 18, dadurch gekennzeichnet, daß dem Precursor Farbmittel zugesetzt werden, wobei das Farbmittel entweder vor dem Auftragen auf das Band im Precursor dispergiert oder gelöst oder die Komponenten über mehrere Düsen getrennt auf das Band aufgetragen werden.

20. Verfahren nach den Ansprüchen 16 bis 19, dadurch gekennzeichnet, daß dem Precursor Netzwerkbildner bzw. Netzwerkwandler in Form löslicher Salze zugesetzt werden.

21. Verfahren nach den Ansprüchen 16 bis 20, dadurch gekennzeichnet, daß zur Glättung der Oberfläche der Matrix dem Precursor Bariumsulfat zugesetzt wird.

22. Verfahren nach den Ansprüchen 16 bis 21, dadurch gekennzeichnet, daß vor dem Aufbringen oder beim Aufbringen auf das endlose Band feine Partikel eines organischen oder anorganischen Pigmentes im Precursor dispergiert werden.

23. Verfahren nach Anspruch 22, dadurch gekennzeichnet, daß die dispergierte Menge an Pigmentpartikeln 0,01 bis 99 Gew.-%, vorzugsweise 1 bis 30 Gew.-%, bezogen auf den Precursor beträgt.

24. Verfahren nach Anspruch 23, dadurch gekennzeichnet, daß die Dispersion aus den Pigmentpartikeln und dem Precursor in einer Perlmühle hergestellt wird.

25. Verfahren nach den Ansprüchen 16 bis 24, dadurch gekennzeichnet, daß der Precursor zusammen mit der wäßrigen Lösung einer Metallverbindung und/oder mit einem löslichen organischen Pigment auf das endlose Band aufgebracht wird.

26. Verfahren nach Anspruch 25, dadurch gekennzeichnet, daß die Metallverbindung durch einen Komplexbildner maskiert ist.

27. Verfahren nach Anspruch 26, dadurch gekennzeichnet, daß der Komplexbildner Zitronensäure, Weinsäure oder EDTA ist.

28. Verfahren nach den Ansprüchen 25 bis 27, dadurch gekennzeichnet, daß der Anteil der Metallverbindungen im Precursor 0,01 bis 50 Gew.-%, vorzugsweise 2 bis 10 Gew.-%, beträgt.

29. Verwendung der Pigmente nach den Ansprüchen 1 bis 15 in Formulierungen wie Lacken, Druckfarben, Kosmetika oder in Kunststoffen oder als Korrosionsschutzmittel.

## Claims

1. Platelet-shaped pigment with high lustre and high opacity or high transparency, consisting of a transparent, inorganic, platelet-shaped matrix prepared by solidification of a liquid precursor, the lustre of the said matrix being obtained by coating it with one or more thin, transparent or semitransparent reflecting layers of metal oxides or of metals, the said pigment being obtainable by
- applying the precursor of the matrix material, in the form of a thin film, to a continuous belt,
- solidifying the liquid film by drying,
- in the solidified film, developing the matrix from the precursor by a chemical reaction,
- subjecting the film solidified by drying to subsequent treatment with an acid,
- then separating the film from the carrier medium and washing it,
- if desired, drying, calcining, grinding and classifying the particles, and
- coating the film particles obtained with one or more layers of metal oxides or of metals.

2. Pigment according to Claim 1, characterized in that the matrix comprises as additional constituent a soluble or insoluble colorant.

3. Pigment according to Claims 1 and 2, characterized in that the transparent inorganic matrix consists of silica, silicate, boron oxide, borate, alumina, aluminate or mixtures thereof.

4. Pigment according to Claims 1 to 3, characterized in that the inorganic matrix has been modified with network formers and/or network modifiers.

5. Pigment according to Claims 1 to 4, characterized in that the inorganic matrix comprises barium sulfate to provide surface smoothness.

6. Pigment according to Claim 5, characterized in that the barium sulfate is present in an amount of from 1% by weight to 50% by weight, preferably from 10% by weight to 25% by weight, in the uncoated substrate.

7. Pigment according to Claims 1 to 6, characterized in that the insoluble colorant is an organic or inorganic pigment.

8. Pigment according to Claim 7, characterized in that the organic pigment is an azo pigment, an anthraquinone pigment, an indigo or thioindigo derivative, a diketopyrrolopyrrole pigment, a perylene pigment or a phthalocyanine pigment.

9. Pigment according to Claim 7, characterized in that the inorganic pigment is carbon black, titanium dioxide, iron oxide, chromium dioxide, cobalt oxide or a mixed oxide.

10. Pigment according to Claims 1 to 6, characterized in that the soluble colorant is a soluble metal compound which leads to a colour-imparting metal oxide, or is a soluble organic pigment.

11. Pigment according to Claim 10, characterized in that the colour-imparting metal oxide is iron oxide, chromium oxide or cobalt oxide.

12. Pigment according to Claim 10, characterized in that the soluble organic pigment is an alkali-soluble hydroxyanthraquinone dye or an acidic azo dye.

13. Pigment according to Claims 1 to 12, characterized in that the metal layer, which is designed as a reflecting layer, consists of silver, gold, palladium, platinum, aluminium, chromium or copper.

14. Pigment according to Claims 1 to 12, characterized in that the metal oxide layer, which is designed as a reflecting layer, consists of chromium oxide, titanium dioxide, iron oxide or a mixture thereof and is used to obtain interference colours.

15. Pigment according to Claims 1 to 14, characterized in that atop the reflecting layer or the matrix there are further layers for specific physical and/or chemical requirements.

16. Process for producing a platelet-shaped pigment with high lustre and high opacity or high transparency, consisting of a transparent, inorganic, platelet-shaped matrix prepared by solidification of a liquid precursor, which matrix is coated with one or more thin, transparent or semitransparent reflecting layers of metal oxides or of metals, wherein
- the precursor of the matrix material is applied, in the form of a thin film, to a continuous belt,
- the liquid film is solidified by drying,
- in the solidified film, the matrix is developed from the precursor by a chemical reaction,
- the resulting layer is then separated from the carrier medium and washed, and
- the particles are, if desired, dried, calcined, ground and classified,
characterized in that the film solidified by drying is then treated with an acid and the resulting film particles are coated with one or more reflecting layers of metal oxides or of metals.

17. Process according to Claim 16, characterized in that solutions of inorganic or organic compounds of the metals aluminium, silicon, potassium or sodium with borates, chlorides, aluminates, poly- and/or metaphosphates, silicates or mixtures thereof are employed as precursors.

18. Process according to Claim 16 and 17, characterized in that the precursor is waterglass.

19. Process according to Claims 16 to 18, characterized in that colorants are added to the precursor, either the colorant being dissolved or dispersed in the precursor before it is applied to the belt, or the components being applied to the belt separately by way of two or more nozzles.

20. Process according to Claims 16 to 19, characterized in that network formers and/or network modifiers in the form of soluble salts are added to the precursor.

21. Process according to Claims 16 to 20, characterized in that barium sulfate is added to the precursor to provide smoothness to the matrix surface.

22. Process according to Claims 16 to 21, characterized in that fine particles of an organic or inorganic pigment are dispersed in the precursor before or while it is applied to the continuous belt.

23. Process according to Claim 22, characterized in that the amount of dispersed pigment particles is from 0.01 to 99% by weight, preferably from 1 to 30% by weight, based on the precursor.

24. Process according to Claim 23, characterized in that the dispersion comprising the pigment particles and the precursor is prepared in a bead mill.

25. Process according to Claims 16 to 24, characterized in that the precursor is applied to the continuous belt together with the aqueous solution of a metal compound and/or with a soluble organic pigment.

26. Process according to Claim 25, characterized in that the metal compound is masked by a complexing agent.

27. Process according to Claim 26, characterized in that the complexing agent is citric acid, tartaric acid or EDTA.

28. Process according to Claims 25 to 27, characterized in that the proportion of metal compounds in the precursor is from 0.01 to 50% by weight, preferably from 2 to 10% by weight.

29. Use of the pigments according to Claims 1 to 15 in formulations such as paints, printing inks or cosmetics or in plastics or as anticorrosion agents.

## Revendications

1. Pigment en paillettes à brillant élevé et grand pouvoir couvrant ou haute transparence, constitué d'une matrice minérale en paillettes transparente, produite par solidification d'un précurseur liquide, qui est recouverte, pour l'obtention du brillant, d'une ou plusieurs minces couches réfléchissantes, transparentes ou semi-transparentes, d'oxydes métalliques ou de métaux, pouvant être obtenu par le fait que
- le précurseur du matériau de la matrice est appliqué sous forme de mince pellicule sur une bande continue,
- la pellicule liquide est solidifiée par séchage,
- dans la pellicule solidifiée, la matrice est développée à partir du précurseur par une réaction chimique,
- la pellicule solidifiée par séchage est ensuite traitée par un acide,
- la pellicule est ensuite séparée du milieu de support et lavée,
- les particules sont éventuellement séchées, calcinées, broyées et classées, et
- les particules de pellicule obtenues sont recouvertes d'une ou plusieurs couches d'oxydes métalliques ou de métaux.

2. Pigment selon la revendication 1, caractérisé en ce que la matrice contient comme composant supplémentaire un colorant soluble ou insoluble.

3. Pigment selon les revendications 1 et 2, caractérisé en ce que la matrice minérale transparente est constituée de dioxyde de silicium, silicate, oxyde de bore, borate, oxyde d'aluminium, aluminate ou de mélanges de ceux-ci.

4. Pigment selon les revendications 1 à 3, caractérisé en ce que la matrice minérale est modifiée avec des formateurs de réseau ou des modificateurs de réseau.

5. Pigment selon les revendications 1 à 4, caractérisé en ce que la matrice minérale contient du sulfate de baryum pour le lissage de la surface.

6. Pigment selon la revendication 5, caractérisé en ce que le sulfate de baryum est contenu, dans le substrat non revêtu, en une proportion de 1 % en poids à 50 % en poids, de préférence de 10 % en poids à 25 % en poids.

7. Pigment selon les revendications 1 à 6, caractérisé en ce que le colorant insoluble est un pigment organique ou minéral.

8. Pigment selon la revendication 7, caractérisé en ce que le pigment organique est un pigment azoique, un pigment anthraquinonique, un dérivé d'indigo ou de thioindigo, un pigment dicétopyrrolo-pyrrole, un pigment pérylène ou un pigment phtalocyanine.

9. Pigment selon la revendication 7, caractérisé en ce que le pigment minéral est le noir de carbone, le dioxyde de titane, l'oxyde de fer, le dioxyde de chrome, l'oxyde de cobalt ou un oxyde mixte.

10. Pigment selon la revendication 1 à 6, caractérisé ence que le colorant soluble est un composé métallique soluble qui conduit à un oxyde métallique chromogène, ou est un pigment organique soluble.

11. Pigment selon la revendication 10, caractérisé en ce que l'oxyde métallique chromogène est l'oxyde de fer, l'oxyde de chrome ou l'oxyde de cobalt.

12. Pigment selon la revendication 10, caractérisé en ce que le pigment organique soluble est un colorant hydroxyanthraquinonique soluble dans une solution alcaline, ou un colorant azoïque acide.

13. Pigment selon les revendications 1 à 12, caractérisé en ce que la couche métallique formée en tant que couche réfléchissante est constituée d'argent, d'or, de palladium, de platine, d'aluminium, de chrome ou de cuivre.

14. Pigment selon les revendications 1 à 12, caractérisé en ce que la couche d'oxyde métallique formée en tant que couche réfléchissante est constituée d'oxyde de chrome, de dioxyde de titane, d'oxyde de fer ou d'un mélange de ceux-ci, et sert à l'obtention de couleurs interférentielles.

15. Pigment selon les revendications 1 à 14, caractérisé en ce que d'autres couches sont appliquées, pour des exigences physiques et/ou chimiques particulières, sur la couche réfléchissante ou la matrice.

16. Procédé pour la préparation d'un pigment en paillettes à brillant élevé et grand pouvoir couvrant ou haute transparence, constitué d'une matrice minérale en paillettes transparente, produite par solidification d'un précurseur liquide, qui est recouverte d'une ou plusieurs minces couches réfléchissantes, transparentes ou semi-transparentes, d'oxydes métalliques ou de métaux, dans lequel
- le précurseur du matériau de la matrice est appliqué sous forme d'une mince pellicule sur une bande continue,
- la pellicule liquide est solidifiée par séchage,
- dans la pellicule solidifiée, la matrice est développée à partir du précurseur par une réaction chimique,
- la couche résultante est ensuite séparée du milieu de support et lavée, et
- les particules sont éventuellement séchées, calcinées, broyées et classées,
caractérisé en ce que la pellicule solidifiée par séchage est ensuite traitée par un acide et les particules de pellicule obtenues sont recouvertes d'une ou plusieurs couches réfléchissantes d'oxydes métalliques ou de métaux.

17. Procédé selon la revendication 16, caractérisé en ce que l'on utilise comme précurseur des solutions de composés organiques ou minéraux des métaux aluminium, silicium, potassium ou sodium avec des borates, chlorures, aluminates, poly- ou métaphosphates, silicates ou des mélanges de ceux-ci.

18. Procédé selon les revendications 16 et 17, caractérisé en ce que le précurseur est un orthosilicate (verre soluble).

19. Procédé selon les revendications 16 à 18, caractérisé en ce que l'on ajoute un colorant au précurseur, soit le colorant étant dissous ou dispersé dans le précurseur avant l'application sur la bande, soit les composants étant appliqués séparément par plusieurs buses sur la bande.

20. Procédé selon les revendications 16 à 19, caractérisé en ce que l'on ajoute au précurseur des formateurs de réseau ou modificateurs de réseau sous forme de sels solubles.

21. Procédé selon les revendications 16 à 20, caractérisé en ce que l'on ajoute au précurseur du sulfate de baryum pour le lissage de la surface de la matrice.

22. Procédé selon les revendications 16 à 21, caractérisé en ce que de fines particules d'un pigment organique ou minéral sont dispersées dans le précurseur, avant l'application ou lors de l'application sur la bande continue.

23. Procédé selon la revendication 22, caractérisé en ce que la quantité dispersée sur les particules de pigment va de 0,01 à 99 % en poids, de préférence de 1 à 30 % en poids, par rapport au précurseur.

24. Procédé selon la revendication 23, caractérisé en ce que la dispersion des particules de pigment et du précurseur est préparée dans un broyeur à billes.

25. Procédé selon les revendications 16 à 24, caractérisé en ce que le précurseur est appliqué sur la bande continue conjointement avec la solution aqueuse d'un composé métallique et/ou avec un pigment organique soluble.

26. Procédé selon la revendication 25, caractérisé en ce que le composé métallique est masqué par un complexant.

27. Procédé selon la revendication 26, caractérisé en ce que le complexant est l'acide citrique, l'acide tartrique ou l'EDTA.

28. Procédé selon les revendication 25 à 27, caractérisé en ce que la proportion des composés métalliques dans le précurseur va de 0,01 à 50 % en poids, de préférence de 2 à 10 % en poids.

29. Utilisation des pigments selon les revendications 1 à 15, dans des formulations telles que des vernis, des encres d'imprimerie, des cosmétiques ou dans des matières plastiques, ou en tant qu'agent de protection contre la corrosion.
